# EUROPEAN PATENT APPLICATION

(11) **EP 3 744 847 A1**
(43) Date of publication of application: **02.12.2020**
(21) Application number: 19740775.2
(22) Date of filing: 21.01.2019
(51) Int. Cl.: C12N 15/49, A61K 38/10, A61K 39/21, A61P 31/18, A61P 37/06, C07K 19/00, C12N 15/86, C12P 21/02, A61K 38/08, C07K 14/155

(54) **SELECTIVE CD8-POSITIVE T CELL-INDUCING VACCINE ANTIGEN**

(30) Priority: 22.01.2018 JP 2018008255
(71) Applicant: Japan as Represented by The Director-General of National Institute of Infectious Diseases, Tokyo 162-8640 (JP); ID Pharma Co., Ltd., Tokyo 102-0071 (JP)
(72) Inventor: MATANO, Tetsuro, Tokyo 162-8640 (JP); ISHII, Hiroshi, Tokyo 162-8640 (JP); INOUE, Makoto, Tokyo 102-0071 (JP); HIRONAKA, Takashi, Tokyo 102-0071 (JP); SHU, Tsugumine, Tokyo 102-0071 (JP); MORI, Toyotaka, Tokyo 102-0071 (JP)
(74) Representative: Schiweck Weinzierl Koch Patentanwälte Partnerschaft mbB
(86) International application number: PCT/JP2019/001607
(87) International publication number: WO 2019/142933

(57) **Abstract**

The present invention provides polypeptides for selectively inducing target antigen-specific CD8-positive T-cell responses. Since induction of human immunodeficiency virus (HIV)-specific CD4-positive T-cell responses by vaccine could promote HIV infection, an HIV vaccine antigen that selectively induces HIV-specific CD8-positive T-cell responses would be useful if obtained. Thus, in the present invention, polypeptide antigens were designed in which 8- to 12-residue amino acid sequences divided from the amino acid sequence of a target antigen protein were connected in an order different from that of the original amino acid sequence. DNA and viral vector vaccines expressing these antigens were tested by inoculation into monkeys. As a result, they were shown to be able to efficiently induce antigen-specific CD8-positive T-cell responses in a selective manner. The instant antigens may be useful as vaccine antigens that induce CD8-positive T cells in a highly selective manner.

SEQUENCE LISTING

<110>  Japan as represented by the Director-General of National
       Institute of Infectious Diseases
       ID Pharma Co., Ltd.

<120>  Vaccine antigens selectively inducing CD8-positive T cells

<130>  D4-A1703P

<150>  JP 2018-008255
<151>  2018-01-22

<160>  54

<170>  PatentIn version 3.5

<210>  1
<211>  60
<212>  PRT
<213>  Artificial Sequence

<220>
<223>  an artificially synthesized sequence

<400>  1

Tyr Pro Val Gln Gln Ile Gly Gly Asn Tyr Val His Leu Pro Leu Ser
1               5                   10                  15

Pro Arg Thr Leu Asn Ala Trp Val Lys Leu Ile Glu Glu Lys Lys Phe
            20                  25                  30

Gly Ala Glu Val Val Pro Gly Phe Gln Ala Leu Ser Glu Gly Cys Thr
        35                  40                  45

Pro Tyr Asp Ile Asn Gln Met Leu Asn Cys Val Gly
    50                  55                  60

<210>  2
<211>  11
<212>  PRT
<213>  Artificial Sequence

<220>
<223>  an artificially synthesized sequence

<400>  2

Tyr Pro Val Gln Gln Ile Gly Gly Asn Tyr Val
1               5                   10

<210>  3
<211>  11
<212>  PRT
<213>  Artificial Sequence

<220>

<223>  an artificially synthesized sequence

<400>  3

His Leu Pro Leu Ser Pro Arg Thr Leu Asn Ala
1               5                   10


<210>  4
<211>  11
<212>  PRT
<213>  Artificial Sequence

<220>
<223>  an artificially synthesized sequence

<400>  4

Trp Val Lys Leu Ile Glu Glu Lys Lys Phe Gly
1               5                   10


<210>  5
<211>  11
<212>  PRT
<213>  Artificial Sequence

<220>
<223>  an artificially synthesized sequence

<400>  5

Ala Glu Val Val Pro Gly Phe Gln Ala Leu Ser
1               5                   10


<210>  6
<211>  11
<212>  PRT
<213>  Artificial Sequence

<220>
<223>  an artificially synthesized sequence

<400>  6

Glu Gly Cys Thr Pro Tyr Asp Ile Asn Gln Met
1               5                   10


<210>  7
<211>  11
<212>  PRT
<213>  Artificial Sequence

<220>
<223>  an artificially synthesized sequence

<400>  7

Leu Ser Pro Arg Thr Leu Asn Ala Trp Val Lys
1               5                   10

<210>  8
<211>  11
<212>  PRT
<213>  Artificial Sequence

<220>
<223>  an artificially synthesized sequence

<400>  8

Glu Lys Lys Phe Gly Ala Glu Val Val Pro Gly
1               5                   10


<210>  9
<211>  11
<212>  PRT
<213>  Artificial Sequence

<220>
<223>  an artificially synthesized sequence

<400>  9

Leu Ser Glu Gly Cys Thr Pro Tyr Asp Ile Asn
1               5                   10


<210>  10
<211>  11
<212>  PRT
<213>  Artificial Sequence

<220>
<223>  an artificially synthesized sequence

<400>  10

Asn Tyr Val His Leu Pro Leu Ser Pro Arg Thr
1               5                   10


<210>  11
<211>  11
<212>  PRT
<213>  Artificial Sequence

<220>
<223>  an artificially synthesized sequence

<400>  11

Pro Arg Thr Leu Asn Ala Trp Val Lys Leu Ile
1               5                   10


<210>  12
<211>  11
<212>  PRT
<213>  Artificial Sequence

<220>

<223>  an artificially synthesized sequence

<400>  12

Lys Leu Ile Glu Glu Lys Lys Phe Gly Ala Glu
1               5                   10


<210>  13
<211>  11
<212>  PRT
<213>  Artificial Sequence

<220>
<223>  an artificially synthesized sequence

<400>  13

Gly Ala Glu Val Val Pro Gly Phe Gln Ala Leu
1               5                   10


<210>  14
<211>  11
<212>  PRT
<213>  Artificial Sequence

<220>
<223>  an artificially synthesized sequence

<400>  14

Gln Ala Leu Ser Glu Gly Cys Thr Pro Tyr Asp
1               5                   10


<210>  15
<211>  11
<212>  PRT
<213>  Artificial Sequence

<220>
<223>  an artificially synthesized sequence

<400>  15

Pro Tyr Asp Ile Asn Gln Met Leu Asn Cys Val
1               5                   10


<210>  16
<211>  8
<212>  PRT
<213>  Artificial Sequence

<220>
<223>  an artificially synthesized sequence

<400>  16

Asn Tyr Val His Leu Pro Leu Ser
1               5

<210>  17
<211>  10
<212>  PRT
<213>  Artificial Sequence

<220>
<223>  an artificially synthesized sequence

<400>  17

Ser Pro Arg Thr Leu Asn Ala Trp Val Lys
1               5                   10


<210>  18
<211>  9
<212>  PRT
<213>  Artificial Sequence

<220>
<223>  an artificially synthesized sequence

<400>  18

Glu Glu Lys Lys Phe Gly Ala Glu Val
1               5


<210>  19
<211>  11
<212>  PRT
<213>  Artificial Sequence

<220>
<223>  an artificially synthesized sequence

<400>  19

Glu Val Val Pro Gly Phe Gln Ala Leu Ser Glu
1               5                   10


<210>  20
<211>  10
<212>  PRT
<213>  Artificial Sequence

<220>
<223>  an artificially synthesized sequence

<400>  20

Val Tyr Tyr Asp Pro Ser Lys Asp Leu Ile
1               5                   10


<210>  21
<211>  510
<212>  PRT
<213>  Simian immunodeficiency virus

<400>  21

Met Gly Val Arg Asn Ser Val Leu Ser Gly Lys Lys Ala Asp Glu Leu
1               5                   10                  15

Glu Lys Ile Arg Leu Arg Pro Asn Gly Lys Lys Lys Tyr Met Leu Lys
            20                  25                  30

His Val Val Trp Ala Ala Asn Glu Leu Asp Arg Phe Gly Leu Ala Glu
        35                  40                  45

Ser Leu Leu Glu Asn Lys Glu Gly Cys Gln Lys Ile Leu Ser Val Leu
    50                  55                  60

Ala Pro Leu Val Pro Thr Gly Ser Glu Asn Leu Lys Ser Leu Tyr Asn
65                  70                  75                  80

Thr Val Cys Val Ile Trp Cys Ile His Ala Glu Glu Lys Val Lys His
                85                  90                  95

Thr Glu Glu Ala Lys Gln Ile Val Gln Arg His Leu Val Val Glu Thr
            100                 105                 110

Gly Thr Thr Glu Thr Met Pro Lys Thr Ser Arg Pro Thr Ala Pro Ser
        115                 120                 125

Ser Gly Arg Gly Gly Asn Tyr Pro Val Gln Gln Ile Gly Gly Asn Tyr
    130                 135                 140

Val His Leu Pro Leu Ser Pro Arg Thr Leu Asn Ala Trp Val Lys Leu
145                 150                 155                 160

Ile Glu Glu Lys Lys Phe Gly Ala Glu Val Val Pro Gly Phe Gln Ala
                165                 170                 175

Leu Ser Glu Gly Cys Thr Pro Tyr Asp Ile Asn Gln Met Leu Asn Cys
            180                 185                 190

Val Gly Asp His Gln Ala Ala Met Gln Ile Ile Arg Asp Ile Ile Asn
        195                 200                 205

Glu Glu Ala Ala Asp Trp Asp Leu Gln His Pro Gln Pro Ala Pro Gln
    210                 215                 220

Gln Gly Gln Leu Arg Glu Pro Ser Gly Ser Asp Ile Ala Gly Thr Thr
225                 230                 235                 240

Ser Ser Val Asp Glu Gln Ile Gln Trp Met Tyr Arg Gln Gln Asn Pro
                245                 250                 255

Ile Pro Val Gly Asn Ile Tyr Arg Arg Trp Ile Gln Leu Gly Leu Gln
            260                 265                 270

Lys Cys Val Arg Met Tyr Asn Pro Thr Asn Ile Leu Asp Val Lys Gln
        275                 280                 285

Gly Pro Lys Glu Pro Phe Gln Ser Tyr Val Asp Arg Phe Tyr Lys Ser
    290                 295                 300

Leu Arg Ala Glu Gln Thr Asp Ala Ala Val Lys Asn Trp Met Thr Gln
305                 310                 315                 320

Thr Leu Leu Ile Gln Asn Ala Asn Pro Asp Cys Lys Leu Val Leu Lys
                325                 330                 335

Gly Leu Gly Val Asn Pro Thr Leu Glu Glu Met Leu Thr Ala Cys Gln
            340                 345                 350

Gly Val Gly Gly Pro Gly Gln Lys Ala Arg Leu Met Ala Glu Ala Leu
        355                 360                 365

Lys Glu Ala Leu Ala Pro Val Pro Ile Pro Phe Ala Ala Ala Gln Gln
    370                 375                 380

Arg Gly Pro Arg Lys Pro Ile Lys Cys Trp Asn Cys Gly Lys Glu Gly
385                 390                 395                 400

His Ser Ala Arg Gln Cys Arg Ala Pro Arg Arg Gln Gly Cys Trp Lys
                405                 410                 415

Cys Gly Lys Met Asp His Val Met Ala Lys Cys Pro Asp Arg Gln Ala
            420                 425                 430

Gly Phe Leu Gly Leu Gly Pro Trp Gly Lys Lys Pro Arg Asn Phe Pro
        435                 440                 445

Met Ala Gln Val His Gln Gly Leu Met Pro Thr Ala Pro Pro Glu Asp
    450                 455                 460

Pro Ala Val Asp Leu Leu Lys Asn Tyr Met Gln Leu Gly Lys Gln Gln
465                 470                 475                 480

Arg Glu Lys Gln Arg Glu Ser Arg Glu Lys Pro Tyr Lys Glu Val Thr
                485                 490                 495

Glu Asp Leu Leu His Leu Asn Ser Leu Phe Gly Gly Asp Gln

500                 505                 510

<210>  22
<211>  214
<212>  PRT
<213>  Simian immunodeficiency virus

<400>  22

Met Glu Glu Glu Lys Arg Trp Ile Ala Val Pro Thr Trp Arg Ile Pro
1               5                   10                  15

Glu Arg Leu Glu Arg Trp His Ser Leu Ile Lys Tyr Leu Lys Tyr Lys
            20                  25                  30

Thr Lys Asp Leu Gln Lys Val Cys Tyr Val Pro His Phe Lys Val Gly
        35                  40                  45

Trp Ala Trp Trp Thr Cys Ser Arg Val Ile Phe Pro Leu Gln Glu Gly
    50                  55                  60

Ser His Leu Glu Val Gln Gly Tyr Trp His Leu Thr Pro Glu Lys Gly
65                  70                  75                  80

Trp Leu Ser Thr Tyr Ala Val Arg Ile Thr Trp Tyr Ser Lys Asn Phe
                85                  90                  95

Trp Thr Asp Val Thr Pro Asn Tyr Ala Asp Ile Leu Leu His Ser Thr
            100                 105                 110

Tyr Phe Pro Cys Phe Thr Ala Gly Glu Val Arg Arg Ala Ile Arg Gly
        115                 120                 125

Glu Gln Leu Leu Ser Cys Cys Arg Phe Pro Arg Ala His Lys Tyr Gln
    130                 135                 140

Val Pro Ser Leu Gln Tyr Leu Ala Leu Lys Val Val Ser Asp Val Arg
145                 150                 155                 160

Ser Gln Gly Glu Asn Pro Thr Trp Lys Gln Trp Arg Arg Asp Asn Arg
                165                 170                 175

Arg Gly Leu Arg Met Ala Lys Gln Asn Ser Arg Gly Asp Lys Gln Arg
            180                 185                 190

Gly Gly Lys Pro Pro Thr Lys Gly Ala Asn Phe Pro Gly Leu Ala Lys
        195                 200                 205

Val Leu Gly Ile Leu Ala

    210

<210>  23
<211>  674
<212>  PRT
<213>  Artificial Sequence

<220>
<223>  an artificially synthesized sequence

<400>  23

Met Ala Tyr Pro Val Gln Gln Ile Gly Gly Asn Tyr Val Ala Gly Ala
1               5                   10                  15

Glu Val Val Pro Gly Phe Gln Ala Leu Ala Ala Met Gln Ile Ile Arg
            20                  25                  30

Asp Ile Ile Asn Glu Ala Pro Ser Gly Ser Asp Ile Ala Gly Thr Thr
        35                  40                  45

Ser Ala Tyr Arg Arg Trp Ile Gln Leu Gly Leu Gln Lys Ala Gln Ser
    50                  55                  60

Tyr Val Asp Arg Phe Tyr Lys Ser Leu Ala Ala Asn Pro Asp Cys Lys
65                  70                  75                  80

Leu Val Leu Lys Gly Ala Gln Lys Ala Arg Leu Met Ala Glu Ala Leu
                85                  90                  95

Lys Ala Asn Tyr Val His Leu Pro Leu Ser Pro Arg Thr Ala Gln Ala
            100                 105                 110

Leu Ser Glu Gly Cys Thr Pro Tyr Asp Ala Ile Asn Glu Glu Ala Ala
        115                 120                 125

Asp Trp Asp Leu Gln Ala Thr Thr Ser Ser Val Asp Glu Gln Ile Gln
    130                 135                 140

Trp Ala Leu Gln Lys Cys Val Arg Met Tyr Asn Pro Thr Ala Lys Ser
145                 150                 155                 160

Leu Arg Ala Glu Gln Thr Asp Ala Ala Ala Leu Lys Gly Leu Gly Val
                165                 170                 175

Asn Pro Thr Leu Glu Ala Ala Leu Lys Glu Ala Leu Ala Pro Val Pro
            180                 185                 190

Ile Ala Pro Arg Thr Leu Asn Ala Trp Val Lys Leu Ile Ala Pro Tyr
        195                 200                 205

Asp Ile Asn Gln Met Leu Asn Cys Val Ala Asp Leu Gln His Pro Gln
    210                 215                 220

Pro Ala Pro Gln Gln Ala Ile Gln Trp Met Tyr Arg Gln Gln Asn Pro
225                 230                 235                 240

Ile Ala Asn Pro Thr Asn Ile Leu Asp Val Lys Gln Gly Ala Asp Ala
                245                 250                 255

Ala Val Lys Asn Trp Met Thr Gln Thr Ala Thr Leu Glu Glu Met Leu
            260                 265                 270

Thr Ala Cys Gln Gly Ala Val Pro Ile Pro Phe Ala Ala Ala Gln Gln
        275                 280                 285

Arg Ala Lys Leu Ile Glu Glu Lys Lys Phe Gly Ala Glu Ala Asn Cys
    290                 295                 300

Val Gly Asp His Gln Ala Ala Met Gln Ala Pro Gln Gln Gly Gln Leu
305                 310                 315                 320

Arg Glu Pro Ser Gly Ala Asn Pro Ile Pro Val Gly Asn Ile Tyr Arg
                325                 330                 335

Arg Ala Lys Gln Gly Pro Lys Glu Pro Phe Gln Ser Tyr Ala Thr Gln
            340                 345                 350

Thr Leu Leu Ile Gln Asn Ala Asn Pro Ala Cys Gln Gly Val Gly Gly
        355                 360                 365

Pro Gly Gln Lys Ala Ala Gln Gln Arg Gly Pro Arg Lys Pro Ile Lys
    370                 375                 380

Cys Ala Met Glu Glu Glu Lys Arg Trp Ile Ala Val Pro Ala Thr Lys
385                 390                 395                 400

Asp Leu Gln Lys Val Cys Tyr Val Pro Ala Ser His Leu Glu Val Gln
                405                 410                 415

Gly Tyr Trp His Leu Ala Trp Thr Asp Val Thr Pro Asn Tyr Ala Asp
            420                 425                 430

Ile Ala Glu Gln Leu Leu Ser Cys Cys Arg Phe Pro Arg Ala Ser Gln
        435                 440                 445

Gly Glu Asn Pro Thr Trp Lys Gln Trp Ala Ala Val Pro Thr Trp Arg

450                 455                 460

Ile Pro Glu Arg Leu Ala Tyr Val Pro His Phe Lys Val Gly Trp Ala
465                 470                 475                 480

Trp Ala Trp His Leu Thr Pro Glu Lys Gly Trp Leu Ser Ala Ala Asp
                485                 490                 495

Ile Leu Leu His Ser Thr Tyr Phe Pro Ala Phe Pro Arg Ala His Lys
            500                 505                 510

Tyr Gln Val Pro Ser Ala Lys Gln Trp Arg Arg Asp Asn Arg Arg Gly
        515                 520                 525

Leu Ala Glu Arg Leu Glu Arg Trp His Ser Leu Ile Lys Ala Trp Ala
    530                 535                 540

Trp Trp Thr Cys Ser Arg Val Ile Phe Ala Trp Leu Ser Thr Tyr Ala
545                 550                 555                 560

Val Arg Ile Thr Trp Ala Tyr Phe Pro Cys Phe Thr Ala Gly Glu Val
                565                 570                 575

Arg Ala Val Pro Ser Leu Gln Tyr Leu Ala Leu Lys Val Ala Arg Gly
            580                 585                 590

Leu Arg Met Ala Lys Gln Asn Ser Arg Ala Leu Ile Lys Tyr Leu Lys
        595                 600                 605

Tyr Lys Thr Lys Asp Ala Val Ile Phe Pro Leu Gln Glu Gly Ser His
    610                 615                 620

Leu Ala Ile Thr Trp Tyr Ser Lys Asn Phe Trp Thr Asp Ala Glu Val
625                 630                 635                 640

Arg Arg Ala Ile Arg Gly Glu Gln Leu Ala Leu Lys Val Val Ser Asp
                645                 650                 655

Val Arg Ser Gln Gly Ala Asn Ser Arg Gly Asp Lys Gln Arg Gly Gly
            660                 665                 670

Lys Ala

<210>  24
<211>  674
<212>  PRT
<213>  Artificial Sequence

<220>
<223>  an artificially synthesized sequence

<400>  24

Met Ala Pro Val Gln Gln Ile Gly Gly Asn Tyr Val His Ala Ala Glu
1               5                   10                  15

Val Val Pro Gly Phe Gln Ala Leu Ser Ala Met Gln Ile Ile Arg Asp
            20                  25                  30

Ile Ile Asn Glu Glu Ala Ser Gly Ser Asp Ile Ala Gly Thr Thr Ser
        35                  40                  45

Ser Ala Arg Arg Trp Ile Gln Leu Gly Leu Gln Lys Cys Ala Ser Tyr
    50                  55                  60

Val Asp Arg Phe Tyr Lys Ser Leu Arg Ala Asn Pro Asp Cys Lys Leu
65                  70                  75                  80

Val Leu Lys Gly Leu Ala Lys Ala Arg Leu Met Ala Glu Ala Leu Lys
                85                  90                  95

Glu Ala Tyr Val His Leu Pro Leu Ser Pro Arg Thr Leu Ala Ala Leu
            100                 105                 110

Ser Glu Gly Cys Thr Pro Tyr Asp Ile Ala Asn Glu Glu Ala Ala Asp
        115                 120                 125

Trp Asp Leu Gln His Ala Thr Ser Ser Val Asp Glu Gln Ile Gln Trp
    130                 135                 140

Met Ala Gln Lys Cys Val Arg Met Tyr Asn Pro Thr Asn Ala Ser Leu
145                 150                 155                 160

Arg Ala Glu Gln Thr Asp Ala Ala Val Ala Lys Gly Leu Gly Val Asn
                165                 170                 175

Pro Thr Leu Glu Glu Ala Leu Lys Glu Ala Leu Ala Pro Val Pro Ile
            180                 185                 190

Pro Ala Arg Thr Leu Asn Ala Trp Val Lys Leu Ile Glu Ala Tyr Asp
        195                 200                 205

Ile Asn Gln Met Leu Asn Cys Val Gly Ala Leu Gln His Pro Gln Pro
    210                 215                 220

Ala Pro Gln Gln Gly Ala Gln Trp Met Tyr Arg Gln Gln Asn Pro Ile

225                 230                 235                 240

Pro Ala Pro Thr Asn Ile Leu Asp Val Lys Gln Gly Pro Ala Ala Ala
                245                 250                 255

Val Lys Asn Trp Met Thr Gln Thr Leu Ala Leu Glu Glu Met Leu Thr
            260                 265                 270

Ala Cys Gln Gly Val Ala Pro Ile Pro Phe Ala Ala Ala Gln Gln Arg
        275                 280                 285

Gly Ala Leu Ile Glu Glu Lys Lys Phe Gly Ala Glu Val Ala Cys Val
    290                 295                 300

Gly Asp His Gln Ala Ala Met Gln Ile Ala Gln Gln Gly Gln Leu Arg
305                 310                 315                 320

Glu Pro Ser Gly Ser Ala Pro Ile Pro Val Gly Asn Ile Tyr Arg Arg
                325                 330                 335

Trp Ala Gln Gly Pro Lys Glu Pro Phe Gln Ser Tyr Val Ala Gln Thr
            340                 345                 350

Leu Leu Ile Gln Asn Ala Asn Pro Asp Ala Gln Gly Val Gly Gly Pro
        355                 360                 365

Gly Gln Lys Ala Arg Ala Gln Arg Gly Pro Arg Lys Pro Ile Lys Cys
    370                 375                 380

Trp Ala Glu Glu Glu Lys Arg Trp Ile Ala Val Pro Thr Ala Lys Asp
385                 390                 395                 400

Leu Gln Lys Val Cys Tyr Val Pro His Ala His Leu Glu Val Gln Gly
                405                 410                 415

Tyr Trp His Leu Thr Ala Thr Asp Val Thr Pro Asn Tyr Ala Asp Ile
            420                 425                 430

Leu Ala Gln Leu Leu Ser Cys Cys Arg Phe Pro Arg Ala Ala Gln Gly
        435                 440                 445

Glu Asn Pro Thr Trp Lys Gln Trp Arg Ala Val Pro Thr Trp Arg Ile
    450                 455                 460

Pro Glu Arg Leu Glu Ala Val Pro His Phe Lys Val Gly Trp Ala Trp
465                 470                 475                 480

Trp Ala His Leu Thr Pro Glu Lys Gly Trp Leu Ser Thr Ala Asp Ile
                485                 490                 495

Leu Leu His Ser Thr Tyr Phe Pro Cys Ala Pro Arg Ala His Lys Tyr
            500                 505                 510

Gln Val Pro Ser Leu Ala Gln Trp Arg Arg Asp Asn Arg Arg Gly Leu
        515                 520                 525

Arg Ala Arg Leu Glu Arg Trp His Ser Leu Ile Lys Tyr Ala Ala Trp
    530                 535                 540

Trp Thr Cys Ser Arg Val Ile Phe Pro Ala Leu Ser Thr Tyr Ala Val
545                 550                 555                 560

Arg Ile Thr Trp Tyr Ala Phe Pro Cys Phe Thr Ala Gly Glu Val Arg
                565                 570                 575

Arg Ala Pro Ser Leu Gln Tyr Leu Ala Leu Lys Val Val Ala Gly Leu
            580                 585                 590

Arg Met Ala Lys Gln Asn Ser Arg Gly Ala Ile Lys Tyr Leu Lys Tyr
        595                 600                 605

Lys Thr Lys Asp Leu Ala Ile Phe Pro Leu Gln Glu Gly Ser His Leu
    610                 615                 620

Glu Ala Thr Trp Tyr Ser Lys Asn Phe Trp Thr Asp Val Ala Val Arg
625                 630                 635                 640

Arg Ala Ile Arg Gly Glu Gln Leu Leu Ala Lys Val Val Ser Asp Val
                645                 650                 655

Arg Ser Gln Gly Glu Ala Ser Arg Gly Asp Lys Gln Arg Gly Gly Lys
            660                 665                 670

Pro Ala



<210>  25
<211>  674
<212>  PRT
<213>  Artificial Sequence

<220>
<223>  an artificially synthesized sequence

<400>  25

Met Ala Val Gln Gln Ile Gly Gly Asn Tyr Val His Leu Ala Glu Val

1               5                   10                  15

Val Pro Gly Phe Gln Ala Leu Ser Glu Ala Gln Ile Ile Arg Asp Ile
            20                  25                  30

Ile Asn Glu Glu Ala Ala Gly Ser Asp Ile Ala Gly Thr Thr Ser Ser
        35                  40                  45

Val Ala Arg Trp Ile Gln Leu Gly Leu Gln Lys Cys Val Ala Tyr Val
    50                  55                  60

Asp Arg Phe Tyr Lys Ser Leu Arg Ala Ala Pro Asp Cys Lys Leu Val
65                  70                  75                  80

Leu Lys Gly Leu Gly Ala Ala Arg Leu Met Ala Glu Ala Leu Lys Glu
                85                  90                  95

Ala Ala Val His Leu Pro Leu Ser Pro Arg Thr Leu Asn Ala Leu Ser
            100                 105                 110

Glu Gly Cys Thr Pro Tyr Asp Ile Asn Ala Glu Glu Ala Ala Asp Trp
        115                 120                 125

Asp Leu Gln His Pro Ala Ser Ser Val Asp Glu Gln Ile Gln Trp Met
    130                 135                 140

Tyr Ala Lys Cys Val Arg Met Tyr Asn Pro Thr Asn Ile Ala Leu Arg
145                 150                 155                 160

Ala Glu Gln Thr Asp Ala Ala Val Lys Ala Gly Leu Gly Val Asn Pro
                165                 170                 175

Thr Leu Glu Glu Met Ala Lys Glu Ala Leu Ala Pro Val Pro Ile Pro
            180                 185                 190

Phe Ala Thr Leu Asn Ala Trp Val Lys Leu Ile Glu Glu Ala Asp Ile
        195                 200                 205

Asn Gln Met Leu Asn Cys Val Gly Asp Ala Gln His Pro Gln Pro Ala
    210                 215                 220

Pro Gln Gln Gly Gln Ala Trp Met Tyr Arg Gln Gln Asn Pro Ile Pro
225                 230                 235                 240

Val Ala Thr Asn Ile Leu Asp Val Lys Gln Gly Pro Lys Ala Ala Val
                245                 250                 255

Lys Asn Trp Met Thr Gln Thr Leu Leu Ala Glu Glu Met Leu Thr Ala
            260                 265                 270

Cys Gln Gly Val Gly Ala Ile Pro Phe Ala Ala Ala Gln Gln Arg Gly
        275                 280                 285

Pro Ala Ile Glu Glu Lys Lys Phe Gly Ala Glu Val Val Ala Val Gly
    290                 295                 300

Asp His Gln Ala Ala Met Gln Ile Ile Ala Gln Gly Gln Leu Arg Glu
305                 310                 315                 320

Pro Ser Gly Ser Asp Ala Ile Pro Val Gly Asn Ile Tyr Arg Arg Trp
                325                 330                 335

Ile Ala Gly Pro Lys Glu Pro Phe Gln Ser Tyr Val Asp Ala Thr Leu
            340                 345                 350

Leu Ile Gln Asn Ala Asn Pro Asp Cys Ala Gly Val Gly Gly Pro Gly
        355                 360                 365

Gln Lys Ala Arg Leu Ala Arg Gly Pro Arg Lys Pro Ile Lys Cys Trp
    370                 375                 380

Asn Ala Glu Glu Lys Arg Trp Ile Ala Val Pro Thr Trp Ala Asp Leu
385                 390                 395                 400

Gln Lys Val Cys Tyr Val Pro His Phe Ala Leu Glu Val Gln Gly Tyr
                405                 410                 415

Trp His Leu Thr Pro Ala Asp Val Thr Pro Asn Tyr Ala Asp Ile Leu
            420                 425                 430

Leu Ala Leu Leu Ser Cys Cys Arg Phe Pro Arg Ala His Ala Gly Glu
        435                 440                 445

Asn Pro Thr Trp Lys Gln Trp Arg Arg Ala Pro Thr Trp Arg Ile Pro
    450                 455                 460

Glu Arg Leu Glu Arg Ala Pro His Phe Lys Val Gly Trp Ala Trp Trp
465                 470                 475                 480

Thr Ala Leu Thr Pro Glu Lys Gly Trp Leu Ser Thr Tyr Ala Ile Leu
                485                 490                 495

Leu His Ser Thr Tyr Phe Pro Cys Phe Ala Arg Ala His Lys Tyr Gln
            500                 505                 510

Val Pro Ser Leu Gln Ala Trp Arg Arg Asp Asn Arg Arg Gly Leu Arg
        515                 520                 525

Met Ala Leu Glu Arg Trp His Ser Leu Ile Lys Tyr Leu Ala Trp Trp
    530                 535                 540

Thr Cys Ser Arg Val Ile Phe Pro Leu Ala Ser Thr Tyr Ala Val Arg
545                 550                 555                 560

Ile Thr Trp Tyr Ser Ala Pro Cys Phe Thr Ala Gly Glu Val Arg Arg
                565                 570                 575

Ala Ala Ser Leu Gln Tyr Leu Ala Leu Lys Val Val Ser Ala Leu Arg
            580                 585                 590

Met Ala Lys Gln Asn Ser Arg Gly Asp Ala Lys Tyr Leu Lys Tyr Lys
        595                 600                 605

Thr Lys Asp Leu Gln Ala Phe Pro Leu Gln Glu Gly Ser His Leu Glu
    610                 615                 620

Val Ala Trp Tyr Ser Lys Asn Phe Trp Thr Asp Val Thr Ala Arg Arg
625                 630                 635                 640

Ala Ile Arg Gly Glu Gln Leu Leu Ser Ala Val Val Ser Asp Val Arg
                645                 650                 655

Ser Gln Gly Glu Asn Ala Arg Gly Asp Lys Gln Arg Gly Gly Lys Pro
            660                 665                 670

Pro Ala

<210>  26
<211>  674
<212>  PRT
<213>  Artificial Sequence

<220>
<223>  an artificially synthesized sequence

<400>  26

Met Ala Gln Gln Ile Gly Gly Asn Tyr Val His Leu Pro Ala Val Val
1               5                   10                  15

Pro Gly Phe Gln Ala Leu Ser Glu Gly Ala Ile Ile Arg Asp Ile Ile
            20                  25                  30

Asn Glu Glu Ala Ala Ala Ser Asp Ile Ala Gly Thr Thr Ser Ser Val
        35                  40                  45

Asp Ala Trp Ile Gln Leu Gly Leu Gln Lys Cys Val Arg Ala Val Asp
    50                  55                  60

Arg Phe Tyr Lys Ser Leu Arg Ala Glu Ala Asp Cys Lys Leu Val Leu
65                  70                  75                  80

Lys Gly Leu Gly Val Ala Arg Leu Met Ala Glu Ala Leu Lys Glu Ala
                85                  90                  95

Leu Ala His Leu Pro Leu Ser Pro Arg Thr Leu Asn Ala Ala Ser Glu
            100                 105                 110

Gly Cys Thr Pro Tyr Asp Ile Asn Gln Ala Glu Ala Ala Asp Trp Asp
        115                 120                 125

Leu Gln His Pro Gln Ala Ser Val Asp Glu Gln Ile Gln Trp Met Tyr
    130                 135                 140

Arg Ala Cys Val Arg Met Tyr Asn Pro Thr Asn Ile Leu Ala Arg Ala
145                 150                 155                 160

Glu Gln Thr Asp Ala Ala Val Lys Asn Ala Leu Gly Val Asn Pro Thr
                165                 170                 175

Leu Glu Glu Met Leu Ala Glu Ala Leu Ala Pro Val Pro Ile Pro Phe
            180                 185                 190

Ala Ala Leu Asn Ala Trp Val Lys Leu Ile Glu Glu Lys Ala Ile Asn
        195                 200                 205

Gln Met Leu Asn Cys Val Gly Asp His Ala His Pro Gln Pro Ala Pro
    210                 215                 220

Gln Gln Gly Gln Leu Ala Met Tyr Arg Gln Gln Asn Pro Ile Pro Val
225                 230                 235                 240

Gly Ala Asn Ile Leu Asp Val Lys Gln Gly Pro Lys Glu Ala Val Lys
                245                 250                 255

Asn Trp Met Thr Gln Thr Leu Leu Ile Ala Glu Met Leu Thr Ala Cys
            260                 265                 270

Gln Gly Val Gly Gly Ala Pro Phe Ala Ala Ala Gln Gln Arg Gly Pro
        275                 280                 285

Arg Ala Glu Glu Lys Lys Phe Gly Ala Glu Val Val Pro Ala Gly Asp
    290                 295                 300

His Gln Ala Ala Met Gln Ile Ile Arg Ala Gly Gln Leu Arg Glu Pro
305                 310                 315                 320

Ser Gly Ser Asp Ile Ala Pro Val Gly Asn Ile Tyr Arg Arg Trp Ile
                325                 330                 335

Gln Ala Pro Lys Glu Pro Phe Gln Ser Tyr Val Asp Arg Ala Leu Leu
            340                 345                 350

Ile Gln Asn Ala Asn Pro Asp Cys Lys Ala Val Gly Gly Pro Gly Gln
        355                 360                 365

Lys Ala Arg Leu Met Ala Gly Pro Arg Lys Pro Ile Lys Cys Trp Asn
    370                 375                 380

Cys Ala Glu Lys Arg Trp Ile Ala Val Pro Thr Trp Arg Ala Leu Gln
385                 390                 395                 400

Lys Val Cys Tyr Val Pro His Phe Lys Ala Glu Val Gln Gly Tyr Trp
                405                 410                 415

His Leu Thr Pro Glu Ala Val Thr Pro Asn Tyr Ala Asp Ile Leu Leu
            420                 425                 430

His Ala Leu Ser Cys Cys Arg Phe Pro Arg Ala His Lys Ala Glu Asn
        435                 440                 445

Pro Thr Trp Lys Gln Trp Arg Arg Asp Ala Thr Trp Arg Ile Pro Glu
    450                 455                 460

Arg Leu Glu Arg Trp Ala His Phe Lys Val Gly Trp Ala Trp Trp Thr
465                 470                 475                 480

Cys Ala Thr Pro Glu Lys Gly Trp Leu Ser Thr Tyr Ala Ala Leu Leu
                485                 490                 495

His Ser Thr Tyr Phe Pro Cys Phe Thr Ala Ala His Lys Tyr Gln Val
            500                 505                 510

Pro Ser Leu Gln Tyr Ala Arg Arg Asp Asn Arg Arg Gly Leu Arg Met
        515                 520                 525

Ala Ala Glu Arg Trp His Ser Leu Ile Lys Tyr Leu Lys Ala Trp Thr
    530                 535                 540

Cys Ser Arg Val Ile Phe Pro Leu Gln Ala Thr Tyr Ala Val Arg Ile
545                 550                 555                 560

Thr Trp Tyr Ser Lys Ala Cys Phe Thr Ala Gly Glu Val Arg Arg Ala
                565                 570                 575

Ile Ala Leu Gln Tyr Leu Ala Leu Lys Val Val Ser Asp Ala Arg Met
            580                 585                 590

Ala Lys Gln Asn Ser Arg Gly Asp Lys Ala Tyr Leu Lys Tyr Lys Thr
        595                 600                 605

Lys Asp Leu Gln Lys Ala Pro Leu Gln Glu Gly Ser His Leu Glu Val
    610                 615                 620

Gln Ala Tyr Ser Lys Asn Phe Trp Thr Asp Val Thr Pro Ala Arg Ala
625                 630                 635                 640

Ile Arg Gly Glu Gln Leu Leu Ser Cys Ala Val Ser Asp Val Arg Ser
                645                 650                 655

Gln Gly Glu Asn Pro Ala Gly Asp Lys Gln Arg Gly Gly Lys Pro Pro
            660                 665                 670

Thr Ala

<210>  27
<211>  674
<212>  PRT
<213>  Artificial Sequence

<220>
<223>  an artificially synthesized sequence

<400>  27

Met Ala Gln Ile Gly Gly Asn Tyr Val His Leu Pro Leu Ala Val Pro
1               5                   10                  15

Gly Phe Gln Ala Leu Ser Glu Gly Cys Ala Ile Arg Asp Ile Ile Asn
            20                  25                  30

Glu Glu Ala Ala Asp Ala Asp Ile Ala Gly Thr Thr Ser Ser Val Asp
        35                  40                  45

Glu Ala Ile Gln Leu Gly Leu Gln Lys Cys Val Arg Met Ala Asp Arg
    50                  55                  60

Phe Tyr Lys Ser Leu Arg Ala Glu Gln Ala Cys Lys Leu Val Leu Lys
65                  70                  75                  80

Gly Leu Gly Val Asn Ala Leu Met Ala Glu Ala Leu Lys Glu Ala Leu
                85                  90                  95

Ala Ala Leu Pro Leu Ser Pro Arg Thr Leu Asn Ala Trp Ala Glu Gly
            100                 105                 110

Cys Thr Pro Tyr Asp Ile Asn Gln Met Ala Ala Ala Asp Trp Asp Leu
        115                 120                 125

Gln His Pro Gln Pro Ala Val Asp Glu Gln Ile Gln Trp Met Tyr Arg
    130                 135                 140

Gln Ala Val Arg Met Tyr Asn Pro Thr Asn Ile Leu Asp Ala Ala Glu
145                 150                 155                 160

Gln Thr Asp Ala Ala Val Lys Asn Trp Ala Gly Val Asn Pro Thr Leu
                165                 170                 175

Glu Glu Met Leu Thr Ala Ala Leu Ala Pro Val Pro Ile Pro Phe Ala
            180                 185                 190

Ala Ala Asn Ala Trp Val Lys Leu Ile Glu Glu Lys Lys Ala Asn Gln
        195                 200                 205

Met Leu Asn Cys Val Gly Asp His Gln Ala Pro Gln Pro Ala Pro Gln
    210                 215                 220

Gln Gly Gln Leu Arg Ala Tyr Arg Gln Gln Asn Pro Ile Pro Val Gly
225                 230                 235                 240

Asn Ala Ile Leu Asp Val Lys Gln Gly Pro Lys Glu Pro Ala Lys Asn
                245                 250                 255

Trp Met Thr Gln Thr Leu Leu Ile Gln Ala Met Leu Thr Ala Cys Gln
            260                 265                 270

Gly Val Gly Gly Pro Ala Phe Ala Ala Ala Gln Gln Arg Gly Pro Arg
        275                 280                 285

Lys Ala Glu Lys Lys Phe Gly Ala Glu Val Val Pro Gly Ala Asp His
    290                 295                 300

Gln Ala Ala Met Gln Ile Ile Arg Asp Ala Gln Leu Arg Glu Pro Ser
305                 310                 315                 320

Gly Ser Asp Ile Ala Ala Val Gly Asn Ile Tyr Arg Arg Trp Ile Gln
                325                 330                 335

Leu Ala Lys Glu Pro Phe Gln Ser Tyr Val Asp Arg Phe Ala Leu Ile
            340                 345                 350

Gln Asn Ala Asn Pro Asp Cys Lys Leu Ala Gly Gly Pro Gly Gln Lys
        355                 360                 365

Ala Arg Leu Met Ala Ala Pro Arg Lys Pro Ile Lys Cys Trp Asn Cys
    370                 375                 380

Gly Ala Lys Arg Trp Ile Ala Val Pro Thr Trp Arg Ile Ala Gln Lys
385                 390                 395                 400

Val Cys Tyr Val Pro His Phe Lys Val Ala Val Gln Gly Tyr Trp His
                405                 410                 415

Leu Thr Pro Glu Lys Ala Thr Pro Asn Tyr Ala Asp Ile Leu Leu His
            420                 425                 430

Ser Ala Ser Cys Cys Arg Phe Pro Arg Ala His Lys Tyr Ala Asn Pro
        435                 440                 445

Thr Trp Lys Gln Trp Arg Arg Asp Asn Ala Trp Arg Ile Pro Glu Arg
    450                 455                 460

Leu Glu Arg Trp His Ala Phe Lys Val Gly Trp Ala Trp Trp Thr Cys
465                 470                 475                 480

Ser Ala Pro Glu Lys Gly Trp Leu Ser Thr Tyr Ala Val Ala Leu His
                485                 490                 495

Ser Thr Tyr Phe Pro Cys Phe Thr Ala Ala His Lys Tyr Gln Val Pro
            500                 505                 510

Ser Leu Gln Tyr Leu Ala Arg Asp Asn Arg Arg Gly Leu Arg Met Ala
        515                 520                 525

Lys Ala Arg Trp His Ser Leu Ile Lys Tyr Leu Lys Tyr Ala Thr Cys
    530                 535                 540

Ser Arg Val Ile Phe Pro Leu Gln Glu Ala Tyr Ala Val Arg Ile Thr
545                 550                 555                 560

Trp Tyr Ser Lys Asn Ala Phe Thr Ala Gly Glu Val Arg Arg Ala Ile

565                 570                 575

Arg Ala Gln Tyr Leu Ala Leu Lys Val Val Ser Asp Val Ala Met Ala
            580                 585                 590

Lys Gln Asn Ser Arg Gly Asp Lys Gln Ala Leu Lys Tyr Lys Thr Lys
        595                 600                 605

Asp Leu Gln Lys Val Ala Leu Gln Glu Gly Ser His Leu Glu Val Gln
    610                 615                 620

Gly Ala Ser Lys Asn Phe Trp Thr Asp Val Thr Pro Asn Ala Ala Ile
625                 630                 635                 640

Arg Gly Glu Gln Leu Leu Ser Cys Cys Ala Ser Asp Val Arg Ser Gln
                645                 650                 655

Gly Glu Asn Pro Thr Ala Asp Lys Gln Arg Gly Gly Lys Pro Pro Thr
            660                 665                 670

Lys Ala

<210>  28
<211>  674
<212>  PRT
<213>  Artificial Sequence

<220>
<223>  an artificially synthesized sequence

<400>  28

Met Ala Ile Gly Gly Asn Tyr Val His Leu Pro Leu Ser Ala Pro Gly
1               5                   10                  15

Phe Gln Ala Leu Ser Glu Gly Cys Thr Ala Arg Asp Ile Ile Asn Glu
            20                  25                  30

Glu Ala Ala Asp Trp Ala Ile Ala Gly Thr Thr Ser Ser Val Asp Glu
        35                  40                  45

Gln Ala Gln Leu Gly Leu Gln Lys Cys Val Arg Met Tyr Ala Arg Phe
    50                  55                  60

Tyr Lys Ser Leu Arg Ala Glu Gln Thr Ala Lys Leu Val Leu Lys Gly
65                  70                  75                  80

Leu Gly Val Asn Pro Ala Met Ala Glu Ala Leu Lys Glu Ala Leu Ala
                85                  90                  95

Pro Ala Pro Leu Ser Pro Arg Thr Leu Asn Ala Trp Val Ala Gly Cys
            100                 105                 110

Thr Pro Tyr Asp Ile Asn Gln Met Leu Ala Ala Asp Trp Asp Leu Gln
        115                 120                 125

His Pro Gln Pro Ala Ala Asp Glu Gln Ile Gln Trp Met Tyr Arg Gln
    130                 135                 140

Gln Ala Arg Met Tyr Asn Pro Thr Asn Ile Leu Asp Val Ala Glu Gln
145                 150                 155                 160

Thr Asp Ala Ala Val Lys Asn Trp Met Ala Val Asn Pro Thr Leu Glu
                165                 170                 175

Glu Met Leu Thr Ala Ala Leu Ala Pro Val Pro Ile Pro Phe Ala Ala
            180                 185                 190

Ala Ala Ala Trp Val Lys Leu Ile Glu Glu Lys Lys Phe Ala Gln Met
        195                 200                 205

Leu Asn Cys Val Gly Asp His Gln Ala Ala Gln Pro Ala Pro Gln Gln
    210                 215                 220

Gly Gln Leu Arg Glu Ala Arg Gln Gln Asn Pro Ile Pro Val Gly Asn
225                 230                 235                 240

Ile Ala Leu Asp Val Lys Gln Gly Pro Lys Glu Pro Phe Ala Asn Trp
                245                 250                 255

Met Thr Gln Thr Leu Leu Ile Gln Asn Ala Leu Thr Ala Cys Gln Gly
            260                 265                 270

Val Gly Gly Pro Gly Ala Ala Ala Ala Gln Gln Arg Gly Pro Arg Lys
        275                 280                 285

Pro Ala Lys Lys Phe Gly Ala Glu Val Val Pro Gly Phe Ala His Gln
    290                 295                 300

Ala Ala Met Gln Ile Ile Arg Asp Ile Ala Leu Arg Glu Pro Ser Gly
305                 310                 315                 320

Ser Asp Ile Ala Gly Ala Gly Asn Ile Tyr Arg Arg Trp Ile Gln Leu
                325                 330                 335

Gly Ala Glu Pro Phe Gln Ser Tyr Val Asp Arg Phe Tyr Ala Ile Gln

            340                 345                 350

Asn Ala Asn Pro Asp Cys Lys Leu Val Ala Gly Pro Gly Gln Lys Ala
        355                 360                 365

Arg Leu Met Ala Glu Ala Arg Lys Pro Ile Lys Cys Trp Asn Cys Gly
    370                 375                 380

Lys Ala Arg Trp Ile Ala Val Pro Thr Trp Arg Ile Pro Ala Lys Val
385                 390                 395                 400

Cys Tyr Val Pro His Phe Lys Val Gly Ala Gln Gly Tyr Trp His Leu
                405                 410                 415

Thr Pro Glu Lys Gly Ala Pro Asn Tyr Ala Asp Ile Leu Leu His Ser
            420                 425                 430

Thr Ala Cys Cys Arg Phe Pro Arg Ala His Lys Tyr Gln Ala Pro Thr
        435                 440                 445

Trp Lys Gln Trp Arg Arg Asp Asn Arg Ala Arg Ile Pro Glu Arg Leu
    450                 455                 460

Glu Arg Trp His Ser Ala Lys Val Gly Trp Ala Trp Trp Thr Cys Ser
465                 470                 475                 480

Arg Ala Glu Lys Gly Trp Leu Ser Thr Tyr Ala Val Arg Ala His Ser
                485                 490                 495

Thr Tyr Phe Pro Cys Phe Thr Ala Gly Ala Lys Tyr Gln Val Pro Ser
            500                 505                 510

Leu Gln Tyr Leu Ala Ala Asp Asn Arg Arg Gly Leu Arg Met Ala Lys
        515                 520                 525

Gln Ala Trp His Ser Leu Ile Lys Tyr Leu Lys Tyr Lys Ala Cys Ser
    530                 535                 540

Arg Val Ile Phe Pro Leu Gln Glu Gly Ala Ala Val Arg Ile Thr Trp
545                 550                 555                 560

Tyr Ser Lys Asn Phe Ala Thr Ala Gly Glu Val Arg Arg Ala Ile Arg
                565                 570                 575

Gly Ala Tyr Leu Ala Leu Lys Val Val Ser Asp Val Arg Ala Ala Lys
            580                 585                 590

Gln Asn Ser Arg Gly Asp Lys Gln Arg Ala Lys Tyr Lys Thr Lys Asp
        595                 600                 605

Leu Gln Lys Val Cys Ala Gln Glu Gly Ser His Leu Glu Val Gln Gly
    610                 615                 620

Tyr Ala Lys Asn Phe Trp Thr Asp Val Thr Pro Asn Tyr Ala Ile Arg
625                 630                 635                 640

Gly Glu Gln Leu Leu Ser Cys Cys Arg Ala Asp Val Arg Ser Gln Gly
                645                 650                 655

Glu Asn Pro Thr Trp Ala Lys Gln Arg Gly Gly Lys Pro Pro Thr Lys
            660                 665                 670

Gly Ala

<210>  29
<211>  674
<212>  PRT
<213>  Artificial Sequence

<220>
<223>  an artificially synthesized sequence

<400>  29

Met Ala Gly Gly Asn Tyr Val His Leu Pro Leu Ser Pro Ala Gly Phe
1               5                   10                  15

Gln Ala Leu Ser Glu Gly Cys Thr Pro Ala Asp Ile Ile Asn Glu Glu
            20                  25                  30

Ala Ala Asp Trp Asp Ala Ala Gly Thr Thr Ser Ser Val Asp Glu Gln
        35                  40                  45

Ile Ala Leu Gly Leu Gln Lys Cys Val Arg Met Tyr Asn Ala Phe Tyr
    50                  55                  60

Lys Ser Leu Arg Ala Glu Gln Thr Asp Ala Leu Val Leu Lys Gly Leu
65                  70                  75                  80

Gly Val Asn Pro Thr Ala Ala Glu Ala Leu Lys Glu Ala Leu Ala Pro
                85                  90                  95

Val Ala Leu Ser Pro Arg Thr Leu Asn Ala Trp Val Lys Ala Cys Thr
            100                 105                 110

Pro Tyr Asp Ile Asn Gln Met Leu Asn Ala Asp Trp Asp Leu Gln His

115                 120                 125

Pro Gln Pro Ala Pro Ala Glu Gln Ile Gln Trp Met Tyr Arg Gln Gln
    130                 135                 140

Asn Ala Met Tyr Asn Pro Thr Asn Ile Leu Asp Val Lys Ala Gln Thr
145                 150                 155                 160

Asp Ala Ala Val Lys Asn Trp Met Thr Ala Asn Pro Thr Leu Glu Glu
                165                 170                 175

Met Leu Thr Ala Cys Ala Ala Pro Val Pro Ile Pro Phe Ala Ala Ala
            180                 185                 190

Gln Ala Trp Val Lys Leu Ile Glu Glu Lys Lys Phe Gly Ala Met Leu
        195                 200                 205

Asn Cys Val Gly Asp His Gln Ala Ala Ala Pro Ala Pro Gln Gln Gly
    210                 215                 220

Gln Leu Arg Glu Pro Ala Gln Gln Asn Pro Ile Pro Val Gly Asn Ile
225                 230                 235                 240

Tyr Ala Asp Val Lys Gln Gly Pro Lys Glu Pro Phe Gln Ala Trp Met
                245                 250                 255

Thr Gln Thr Leu Leu Ile Gln Asn Ala Ala Thr Ala Cys Gln Gly Val
            260                 265                 270

Gly Gly Pro Gly Gln Ala Ala Ala Gln Gln Arg Gly Pro Arg Lys Pro
        275                 280                 285

Ile Ala Lys Phe Gly Ala Glu Val Val Pro Gly Phe Gln Ala Gln Ala
    290                 295                 300

Ala Met Gln Ile Ile Arg Asp Ile Ile Ala Arg Glu Pro Ser Gly Ser
305                 310                 315                 320

Asp Ile Ala Gly Thr Ala Asn Ile Tyr Arg Arg Trp Ile Gln Leu Gly
                325                 330                 335

Leu Ala Pro Phe Gln Ser Tyr Val Asp Arg Phe Tyr Lys Ala Gln Asn
            340                 345                 350

Ala Asn Pro Asp Cys Lys Leu Val Leu Ala Pro Gly Gln Lys Ala Arg
        355                 360                 365

Leu Met Ala Glu Ala Ala Lys Pro Ile Lys Cys Trp Asn Cys Gly Lys
    370                 375                 380

Glu Ala Trp Ile Ala Val Pro Thr Trp Arg Ile Pro Glu Ala Val Cys
385                 390                 395                 400

Tyr Val Pro His Phe Lys Val Gly Trp Ala Gly Tyr Trp His Leu Thr
                405                 410                 415

Pro Glu Lys Gly Trp Ala Asn Tyr Ala Asp Ile Leu Leu His Ser Thr
            420                 425                 430

Tyr Ala Cys Arg Phe Pro Arg Ala His Lys Tyr Gln Val Ala Thr Trp
        435                 440                 445

Lys Gln Trp Arg Arg Asp Asn Arg Arg Ala Ile Pro Glu Arg Leu Glu
    450                 455                 460

Arg Trp His Ser Leu Ala Val Gly Trp Ala Trp Trp Thr Cys Ser Arg
465                 470                 475                 480

Val Ala Lys Gly Trp Leu Ser Thr Tyr Ala Val Arg Ile Ala Ser Thr
                485                 490                 495

Tyr Phe Pro Cys Phe Thr Ala Gly Glu Ala Tyr Gln Val Pro Ser Leu
            500                 505                 510

Gln Tyr Leu Ala Leu Ala Asn Arg Arg Gly Leu Arg Met Ala Lys Gln
        515                 520                 525

Asn Ala His Ser Leu Ile Lys Tyr Leu Lys Tyr Lys Thr Ala Ser Arg
    530                 535                 540

Val Ile Phe Pro Leu Gln Glu Gly Ser Ala Val Arg Ile Thr Trp Tyr
545                 550                 555                 560

Ser Lys Asn Phe Trp Ala Ala Gly Glu Val Arg Arg Ala Ile Arg Gly
                565                 570                 575

Glu Ala Leu Ala Leu Lys Val Val Ser Asp Val Arg Ser Ala Lys Gln
            580                 585                 590

Asn Ser Arg Gly Asp Lys Gln Arg Gly Ala Tyr Lys Thr Lys Asp Leu
        595                 600                 605

Gln Lys Val Cys Tyr Ala Glu Gly Ser His Leu Glu Val Gln Gly Tyr
    610                 615                 620

Trp Ala Asn Phe Trp Thr Asp Val Thr Pro Asn Tyr Ala Ala Arg Gly
625                 630                 635                 640

Glu Gln Leu Leu Ser Cys Cys Arg Phe Ala Val Arg Ser Gln Gly Glu
                645                 650                 655

Asn Pro Thr Trp Lys Ala Gln Arg Gly Gly Lys Pro Pro Thr Lys Gly
            660                 665                 670

Ala Ala

<210>  30
<211>  674
<212>  PRT
<213>  Artificial Sequence

<220>
<223>  an artificially synthesized sequence

<400>  30

Met Ala Gly Asn Tyr Val His Leu Pro Leu Ser Pro Arg Ala Phe Gln
1               5                   10                  15

Ala Leu Ser Glu Gly Cys Thr Pro Tyr Ala Ile Ile Asn Glu Glu Ala
            20                  25                  30

Ala Asp Trp Asp Leu Ala Gly Thr Thr Ser Ser Val Asp Glu Gln Ile
        35                  40                  45

Gln Ala Gly Leu Gln Lys Cys Val Arg Met Tyr Asn Pro Ala Tyr Lys
    50                  55                  60

Ser Leu Arg Ala Glu Gln Thr Asp Ala Ala Val Leu Lys Gly Leu Gly
65                  70                  75                  80

Val Asn Pro Thr Leu Ala Glu Ala Leu Lys Glu Ala Leu Ala Pro Val
                85                  90                  95

Pro Ala Ser Pro Arg Thr Leu Asn Ala Trp Val Lys Leu Ala Thr Pro
            100                 105                 110

Tyr Asp Ile Asn Gln Met Leu Asn Cys Ala Trp Asp Leu Gln His Pro
        115                 120                 125

Gln Pro Ala Pro Gln Ala Gln Ile Gln Trp Met Tyr Arg Gln Gln Asn
    130                 135                 140

Pro Ala Tyr Asn Pro Thr Asn Ile Leu Asp Val Lys Gln Ala Thr Asp
145                 150                 155                 160

Ala Ala Val Lys Asn Trp Met Thr Gln Ala Pro Thr Leu Glu Glu Met
                165                 170                 175

Leu Thr Ala Cys Gln Ala Pro Val Pro Ile Pro Phe Ala Ala Ala Gln
            180                 185                 190

Gln Ala Val Lys Leu Ile Glu Glu Lys Lys Phe Gly Ala Ala Leu Asn
        195                 200                 205

Cys Val Gly Asp His Gln Ala Ala Met Ala Ala Pro Gln Gln Gly Gln
    210                 215                 220

Leu Arg Glu Pro Ser Ala Gln Asn Pro Ile Pro Val Gly Asn Ile Tyr
225                 230                 235                 240

Arg Ala Val Lys Gln Gly Pro Lys Glu Pro Phe Gln Ser Ala Met Thr
                245                 250                 255

Gln Thr Leu Leu Ile Gln Asn Ala Asn Ala Ala Cys Gln Gly Val Gly
            260                 265                 270

Gly Pro Gly Gln Lys Ala Ala Gln Gln Arg Gly Pro Arg Lys Pro Ile
        275                 280                 285

Lys Ala Phe Gly Ala Glu Val Val Pro Gly Phe Gln Ala Ala Ala Ala
    290                 295                 300

Met Gln Ile Ile Arg Asp Ile Ile Asn Ala Glu Pro Ser Gly Ser Asp
305                 310                 315                 320

Ile Ala Gly Thr Thr Ala Ile Tyr Arg Arg Trp Ile Gln Leu Gly Leu
                325                 330                 335

Gln Ala Phe Gln Ser Tyr Val Asp Arg Phe Tyr Lys Ser Ala Asn Ala
            340                 345                 350

Asn Pro Asp Cys Lys Leu Val Leu Lys Ala Gly Gln Lys Ala Arg Leu
        355                 360                 365

Met Ala Glu Ala Leu Ala Pro Ile Lys Cys Trp Asn Cys Gly Lys Glu
    370                 375                 380

Gly Ala Ile Ala Val Pro Thr Trp Arg Ile Pro Glu Arg Ala Cys Tyr
385                 390                 395                 400

Val Pro His Phe Lys Val Gly Trp Ala Ala Tyr Trp His Leu Thr Pro
                405                 410                 415

Glu Lys Gly Trp Leu Ala Tyr Ala Asp Ile Leu Leu His Ser Thr Tyr
            420                 425                 430

Phe Ala Arg Phe Pro Arg Ala His Lys Tyr Gln Val Pro Ala Trp Lys
        435                 440                 445

Gln Trp Arg Arg Asp Asn Arg Arg Gly Ala Pro Glu Arg Leu Glu Arg
    450                 455                 460

Trp His Ser Leu Ile Ala Gly Trp Ala Trp Trp Thr Cys Ser Arg Val
465                 470                 475                 480

Ile Ala Gly Trp Leu Ser Thr Tyr Ala Val Arg Ile Thr Ala Thr Tyr
                485                 490                 495

Phe Pro Cys Phe Thr Ala Gly Glu Val Ala Gln Val Pro Ser Leu Gln
            500                 505                 510

Tyr Leu Ala Leu Lys Ala Arg Arg Gly Leu Arg Met Ala Lys Gln Asn
        515                 520                 525

Ser Ala Ser Leu Ile Lys Tyr Leu Lys Tyr Lys Thr Lys Ala Arg Val
    530                 535                 540

Ile Phe Pro Leu Gln Glu Gly Ser His Ala Arg Ile Thr Trp Tyr Ser
545                 550                 555                 560

Lys Asn Phe Trp Thr Ala Gly Glu Val Arg Arg Ala Ile Arg Gly Glu
                565                 570                 575

Gln Ala Ala Leu Lys Val Val Ser Asp Val Arg Ser Gln Ala Gln Asn
            580                 585                 590

Ser Arg Gly Asp Lys Gln Arg Gly Gly Ala Lys Thr Lys Asp Leu Gln
        595                 600                 605

Lys Val Cys Tyr Val Ala Gly Ser His Leu Glu Val Gln Gly Tyr Trp
    610                 615                 620

His Ala Phe Trp Thr Asp Val Thr Pro Asn Tyr Ala Asp Ala Gly Glu
625                 630                 635                 640

Gln Leu Leu Ser Cys Cys Arg Phe Pro Ala Arg Ser Gln Gly Glu Asn
                645                 650                 655

Pro Thr Trp Lys Gln Ala Arg Gly Gly Lys Pro Pro Thr Lys Gly Ala
            660                 665                 670

Asn Ala

<210>  31
<211>  2025
<212>  DNA
<213>  Artificial Sequence

<220>
<223>  an artificially synthesized sequence

<400>  31
atggcctacc ctgtgcagca gatcggaggc aattacgtgg ccggagctga ggtggtgcca      60

ggatttcagg ctctggccgc tatgcagatc atcagggaca tcatcaatga ggccccaagc     120

ggatctgaca tcgccggcac cacctctgcc tataggagat ggatccagct gggcctgcaa     180

aaggcccaga gctacgtgga taggttttat aaatccctgg ctgccaatcc tgactgcaaa     240

ctggtgctga aaggagctca gaaagctcgg ctgatggctg aggccctcaa ggctaattat     300

gtgcatctgc cactgtctcc caggacagcc caggccctgt ccgagggatg caccccttat     360

gacgccatca acgaggaggc cgctgactgg gatctccagg ctaccacatc ttccgtggat     420

gagcagatcc aatgggccct gcagaagtgc gtgagaatgt acaaccctac agctaaatct     480

ctgagagctg agcagaccga cgctgctgcc ctgaagggac tgggcgtgaa tccaacactc     540

gaagctgccc tgaaggaggc tctggctcct gtgcccatcg ctccccgcac actgaacgcc     600

tgggtgaagc tcatcgcccc ttacgacatc aaccagatgc tgaattgtgt ggctgatctg     660

cagcatccac agccagctcc tcaacaggcc atccagtgga tgtaccgcca gcagaaccct     720

atcgccaacc ccacaaatat cctggacgtg aaacaaggcg ccgacgccgc tgtgaagaac     780

tggatgaccc aaacagccac actggaggag atgctgaccg cttgtcaagg agccgtgcca     840

atccccttcg ccgctgccca gcaaagggcc aagctgatcg aggagaaaaa gttcggagcc     900

gaggccaatt gcgtggggga tcaccaggcc gccatgcaag cccctcagca gggacagctg     960

agggagccat ccggagctaa cccaatcccc gtgggaaata tctatagaag agccaaacag    1020

ggccctaagg agccattcca gtcctacgcc acccagacac tgctgatcca gaacgccaac    1080

cctgcctgtc agggagtggg aggacctgga cagaaggctg ctcagcagag gggaccaagg    1140

aaacctatca aatgcgccat ggaggaggag aaaaggtgga tcgccgtccc agccaccaag    1200

gacctgcaga aagtgtgcta tgtgcccgcc tcccacctgg aggtgcaggg atactggcac    1260

ctggcctgga ccgacgtgac acccaattac gctgacatcg ctgagcagct gctgtcctgc    1320

tgtaggtttc ccagagccag ccagggcgag aacccaacct ggaagcaatg ggcagccgtg 1380

ccaacctgga gaatccccga gaggctggcc tacgtgccac atttcaaagt ggggtgggca 1440

tgggcctggc atctgacccc tgagaaagga tggctcagcg ccgctgatat cctgctgcac 1500

tctacctact tccccgcctt tcctagagcc cataaatacc aggtgcccag cgccaagcag 1560

tggcggcgcg acaataggag aggcctggcc gagcggctgg agaggtggca ctccctgatt 1620

aaggcctggg cttggtggac atgttctaga gtcatcttcg cctggctgag cacctacgcc 1680

gtgagaatca cctgggccta tttcccttgc tttacagccg gcgaggtcag ggccgtgcca 1740

agcctgcagt atctggctct gaaagtggcc agaggactgc gcatggccaa acagaactct 1800

agggctctga tcaagtacct gaagtataaa accaaagacg ccgtgatctt tccactgcag 1860

gagggatctc acctcgccat cacatggtat tctaagaact tctggacaga cgctgaggtg 1920

aggagagcta tcagggggga gcaactggcc ctgaaggtgg tgtccgatgt gcggagccaa 1980

ggcgctaact ccagggcgca taagcagagg ggaggcaaag cctga 2025

<210> 32
<211> 2025
<212> DNA
<213> Artificial Sequence

<220>
<223> an artificially synthesized sequence

<400> 32
atggcccctg tgcagcagat cggaggcaat tatgtccatg ccgctgaggt ggtgccagga 60

tttcaggccc tctccgccat gcagatcatc agggacatca tcaacgaaga ggccagcgga 120

tctgacatcg ccggcaccac atcctctgcc aggagatgga tccagctggg cctgcagaaa 180

tgcgccagct acgtggatag gttttataaa tctctcagag ccaatcctga ctgcaaactg 240

gtgctgaagg gcctggccaa agctcggctg atggctgagg ccctgaaaga ggcttatgtg 300

catctgccac tgtctccccg cactctggcc gccctgtccg agggatgcac cccttacgat 360

atcgccaacg aggaggccgc tgactgggat ctgcaacatg ctacatcttc cgtggatgag 420

cagatccaat ggatggccca gaagtgcgtg agaatgtaca accccactaa tgcctctctg 480

agagctgagc agaccgacgc cgcagtggcc aagggactgg gcgtgaatcc aacactggaa 540

gaagccctga aggaggctct ggctcctgtg ccaattcccg ctcgcacact gaacgcctgg 600

gtgaagctca tcgaggctta cgacatcaac cagatgctga attgcgtcgg ggccctgcag 660

catccacagc cagctcctca gcaaggagct cagtggatgt accgccagca gaacccaatt 720

cccgccccca caaatatcct ggacgtgaaa cagggacctg ccgccgctgt gaagaactgg 780

atgacccaga ctctggctct ggaggagatg ctgaccgctt gtcagggcgt ggccccaatc 840

cccttcgccg ctgcccagca gagaggagcc ctgatcgagg agaaaaagtt cggagctgaa 900

gtcgcctgcg tgggggatca ccaggccgct atgcaaatcg cccagcaggg acagctgagg 960

gagccaagcg gctctgctcc aatccccgtg ggaaatatct ataggaggtg ggcccagggc 1020

cctaaggagc cattccagag ctatgtggct cagacactgc tgatccagaa cgccaatccc 1080

gacgcccagg gagtgggagg acctggacag aaagcacggg cccagagggg accaaggaaa 1140

cctatcaagt gttgggctga ggaggagaaa aggtggatcg ccgtgcctac cgctaaggac 1200

ctgcagaaag tgtgctacgt cccacatgcc cacctggagg tgcagggata ctggcatctc 1260

accgccaccg acgtgacacc caattacgct gatattctcg cccagctgct gtcctgctgt 1320

aggtttccta gggccgctca gggcgagaac ccaacctgga agcaatggag ggccgtgcca 1380

acctggagaa tccccgagag gctggaagcc gtgccacatt tcaaagtggg gtgggcctgg 1440

tgggctcatc tgacccctga gaaaggatgg ctgtccaccg cagatatcct gctgcactct 1500

acctatttcc catgcgcccc tagagcccat aaataccagg tgccatccct ggcccagtgg 1560

cggcgcgaca ataggagagg actccgcgcc cggctggaga ggtggcactc cctgatcaaa 1620

tacgccgctt ggtggacatg ttctagagtg atcttcccag ccctgagcac ctacgccgtg 1680

agaatcacct ggtacgcctt cccttgcttt acagccggcg aggtgcggag agccccaagc 1740

ctgcagtatc tggctctgaa ggtcgtggcc ggactgcgca tggccaaaca gaactccaga 1800

ggcgccatca agtacctgaa gtataaaacc aaggatctgg ccatctttcc actgcaggag 1860

ggatcccatc tggaagctac atggtattct aagaacttct ggacagacgt cgccgtgagg 1920

agagctatca ggggggagca gctcctggct aaggtggtgt ccgatgtgcg gagccaggga 1980

gaggcttcca ggggcgataa gcagagggga gggaagcccg cctga 2025

<210> 33
<211> 2025
<212> DNA
<213> Artificial Sequence

<220>
<223> an artificially synthesized sequence

<400> 33
atggccgtgc agcagatcgg aggcaattat gtgcacctgg ccgaggtggt gccaggattt 60

caggccctgt ctgaggccca gatcatcagg gacatcatca acgaggaagc cgccggatct 120

gacatcgccg gcaccacatc tagcgtggcc agatggatcc agctgggcct gcagaagtgt 180

gtggcttacg tggataggtt ttataaatct ctgagggctg cccctgactg caaactggtg 240

ctgaagggac tcggcgccgc tcggctgatg gctgaggccc tgaaggaagc tgctgtgcat 300

ctgccactgt ctccccgcac actcaacgcc ctgtccgagg gatgcacccc ttacgacatt 360

aacgctgagg aggccgctga ctgggatctg cagcacccag cctcttccgt ggatgagcag 420

atccagtgga tgtatgccaa gtgcgtgaga atgtacaacc ccacaaacat cgccctgaga 480

gctgagcaga ccgacgccgc tgtcaaggct ggactgggcg tgaatccaac actggaggaa 540

atggccaagg aggctctggc tcctgtgcca atcccattcg ccacactgaa cgcctgggtg 600

aagctgattg aggaagccga catcaaccag atgctgaatt gcgtgggcga tgctcagcat 660

ccacagccag ctcctcagca ggggcaggcc tggatgtacc gccagcagaa cccaatccct 720

gtggccacaa atatcctgga cgtgaaacag ggccccaaag ccgctgtgaa gaactggatg 780

acccagactc tgctcgccga ggagatgctg accgcttgtc agggagtcgg agccatcccc 840

ttcgccgctg cccagcagag gggcccagcc atcgaggaga aaaagttcgg agctgaggtc 900

gtggctgtgg gggatcacca ggccgctatg cagattatcg ctcagggaca gctgagggag 960

ccaagcggat ccgacgccat ccccgtggga aatatctata ggaggtggat tgccggccct 1020

aaggagccat tccagagcta cgtcgatgcc acactgctga tccagaacgc caatcctgat 1080

tgcgccggag tgggaggacc tggacagaaa gctaggctgg ctaggggacc aaggaaacct 1140

atcaagtgtt ggaatgccga ggagaaaagg tggatcgccg tgccaacatg ggccgacctg 1200

cagaaagtgt gctacgtgcc acacttcgct ctggaggtgc agggatactg gcatctgaca 1260

cctgccgacg tgacacccaa ttacgctgat atcctcctgg cactgctgtc ctgctgtagg 1320

tttcctagag ctcatgccgg cgagaaccca acctggaagc agtggaggcg cgccccaacc 1380

tggagaatcc ccgagcggct ggaaagggct ccacatttca aagtggggtg ggcttggtgg 1440

accgccctga cccctgagaa aggatggctg agcacatacg ccatcctgct gcactctacc 1500

tatttccctt gttttgccag agcccataaa taccaggtgc caagcctcca ggcttggcgg 1560

cgcgacaata ggagaggact gcggatggct ctggagaggt ggcactccct gatcaagtat 1620

ctcgcctggt ggacatgttc tagagtgatc tttcctctgg ctagcaccta cgccgtgaga 1680

atcacatggt actctgcccc ttgctttaca gccggcgagg tgaggagggc tgcaagcctg 1740

cagtatctgg ctctgaaggt ggtctccgcc ctgcgcatgg ccaaacagaa ctccagggga 1800

gatgccaagt acctgaagta taaaaccaag gacctccagg catttccact gcaggaggga 1860

tcccacctgg aagtggcctg gtattctaag aacttctgga ccgacgtcac agccaggaga 1920

gctatcaggg gggagcagct gctctccgcc gtggtgtccg atgtgcggag ccagggcgaa 1980

aacgccaggg gcgataagca gaggggaggg aaacctcctg cttga 2025

<210> 34
<211> 2025
<212> DNA
<213> Artificial Sequence

<220>
<223> an artificially synthesized sequence

<400> 34
atggcccagc agatcggagg caattatgtg catctcccag ccgtggtgcc aggatttcag 60

gccctgtccg aaggagccat catcagggac atcatcaacg aggaggctgc tgcctctgac 120

atcgccggca ccacatcttc cgtcgatgcc tggatccagc tgggcctgca gaagtgtgtg 180

agggccgtgg ataggtttta taaatctctg agagccgagg ccgactgcaa actggtgctg 240

aagggactgg gagtggcccg gctgatggct gaggccctga aggaggccct ggcccatctg 300

ccactgtctc cccgcacact gaatgccgcc tccgagggat gcacccctta cgacatcaat 360

caggccgagg ccgctgactg ggatctgcag catccccagg cttccgtgga tgagcagatc 420

cagtggatgt atcgcgcctg cgtgagaatg tacaacccca caaatattct ggctagagct 480

gagcagaccg acgccgctgt gaaaaacgcc ctgggcgtga atccaacact ggaggaaatg 540

ctcgccgagg ctctggctcc tgtgccaatc ccctttgccg ctctgaacgc ctgggtgaag 600

ctgatcgagg aaaaagccat caaccagatg ctgaattgcg tgggggacca cgcccatcca 660

cagccagctc ctcagcaggg acaactggca atgtaccgcc agcagaaccc aatccccgtc 720

ggggccaata tcctggacgt gaaacagggc cctaaagagg ctgtgaagaa ctggatgacc 780

cagacactgc tcatcgccga gatgctgacc gcttgtcagg gagtgggcgg agctcccttc 840

gccgctgccc agcagagggg acccagggct gaggagaaaa agttcggagc tgaggtggtc 900

ccagctgggg atcaccaggc cgctatgcag atcattcggg ccggacagct gagggagcca 960

agcggatctg atatcgctcc cgtgggaaat atctatagga gatggattca ggctcctaag 1020

gagccattcc agagctacgt ggacagggcc ctgctgatcc agaacgccaa tcctgactgt 1080

aaagccgtgg gaggacctgg acagaaagct cggctcatgg ccggaccaag gaaacctatc 1140

aagtgctgga attgtgccga gaaaaggtgg atcgccgtgc caacatggag ggctctgcag 1200

aaagtgtgct acgtgccaca ttttaaagct gaggtgcagg gatactggca tctgacccca 1260

gaggccgtga cacccaatta cgctgatatc ctgctccacg ctctgtcctg ctgtaggttt 1320

cctagagccc acaaggccga gaacccaacc tggaagcagt ggcggcggga cgccacctgg 1380

agaatccccg agcggctgga gagatgggcc catttcaaag tggggtgggc ttggtggacc 1440

tgtgccaccc ctgagaaagg atggctgagc acctatgctg ctctgctgca ctctacctat 1500

ttcccttgct tcacagccgc ccataaatac caggtgccaa gcctgcaata tgcccggcgc 1560

gacaatagga gaggactgcg gatggcagcc gagaggtggc actccctgat caagtacctc 1620

aaggcttgga catgttctag agtgatcttt cctctgcaag ccacctacgc cgtgagaatc 1680

acatggtatt ccaaggcctg ctttacagcc ggcgaggtga ggagagccat cgccctgcag 1740

tatctggctc tgaaggtggt gtctgatgcc cgcatggcca aacagaactc caggggcgac 1800

aaggcatacc tgaagtataa aaccaaggac ctgcaaaaag ccccactgca ggagggatcc 1860

cacctggagg tccaggccta ttctaagaac ttctggaccg acgtgactcc cgccagagct 1920

atcagggggg agcagctgct gtcttgcgcc gtgtccgatg tgcggagcca gggcgagaat 1980

ccagccggcg ataagcagag gggagggaaa cccccaacag cctga 2025

<210> 35
<211> 2025
<212> DNA
<213> Artificial Sequence

<220>
<223> an artificially synthesized sequence

<400> 35
atggcccaga tcggaggcaa ttatgtgcat ctgcctctgg ccgtgccagg atttcaggcc 60

ctgtccgagg ggtgcgccat cagggacatc atcaacgagg aggccgccga cgccgacatc 120

gccggcacca catcttccgt ggacgaggcc atccagctgg gcctgcagaa gtgcgtgagg 180

atggctgata ggttttataa atctctgaga gctgaacagg cctgcaaact ggtgctgaag 240

ggactgggcg tcaatgccct gatggctgag gccctgaagg aggctctcgc tgccctgcca 300

ctgtctcccc gcacactgaa cgcttgggcc gagggatgca ccccttacga catcaaccaa 360

atggccgccg ctgactggga tctgcagcat ccccagcctg ccgtggatga gcagatccag 420

tggatgtacc ggcaggctgt gagaatgtac aaccccacaa atatcctcga cgctgctgag 480

cagaccgacg ccgctgtgaa gaattgggct ggcgtgaatc caacactgga ggagatgctc 540

accgccgctc tggctcctgt gccaatcccc ttcgctgctg ctaacgcctg ggtgaagctg 600

atcgaggaga agaaagccaa ccagatgctg aattgcgtgg gggatcatca agccccacag 660

ccagctcctc agcagggaca gctcagggcc taccgccagc agaacccaat ccccgtgggc 720

aatgctatcc tggacgtgaa acagggccct aaggaaccag ccaagaactg gatgacccag 780

acactgctga ttcaggccat gctgaccgct tgtcagggag tgggaggccc tgccttcgcc 840

gctgcccagc agaggggacc acggaaggcc gagaaaaagt tcggagctga ggtggtgcct 900

ggagccgatc accaggccgc tatgcagatc atcagagacg cccagctgag ggagccaagc 960

ggatctgaca ttgccgccgt gggaaatatc tataggagat ggatccaact ggctaaggag 1020

ccattccaga gctacgtgga tagatttgcc ctgatccaga acgccaatcc tgactgcaag 1080

ctggcaggag gacctggaca gaaagctcgg ctcatggccg ctccaaggaa acctatcaag 1140

tgctggaact gcggcgccaa aaggtggatc gccgtgccaa cctggaggat cgcccagaaa 1200

gtgtgctacg tgccacattt caaggtggcc gtgcagggat actggcatct gacccctgaa 1260

aaagctacac ccaattacgc tgatatcctg ctgcattctg cctcctgctg taggtttcct 1320

agagcccata agtacgccaa cccaacctgg aagcagtggc ggcgcgataa tgcatggaga 1380

atccccgagc ggctggagag atggcatgcc ttcaaagtgg ggtgggcttg gtggacatgc 1440

tctgctcctg agaaaggatg gctgagcacc tacgctgtgg ctctgcactc tacctatttc 1500

ccttgcttta ccgccgccca taaataccag gtgccaagcc tgcagtacct ggcccgcgac 1560

aataggagag gactgcgcat ggctaaagca aggtggcact ccctgatcaa gtacctgaaa 1620

tatgccacat gttctagagt gatctttcca ctccaggaag cctacgccgt gagaatcaca 1680

tggtattcta aaaacgcctt tacagccggc gaggtgagga gagctattag ggcccagtat 1740

ctggctctga aggtggtgtc cgacgtggcc atggccaaac agaactccag gggcgataaa 1800

caggctctga agtataaaac caaggacctg cagaaggtgg ctctgcagga gggatcccac 1860

ctggaggtgc aaggagcttc taagaacttc tggaccgacg tgacacctaa tgccgctatc 1920

aggggggagc agctgctgtc ctgttgtgcc tccgatgtgc ggagccaggg cgagaaccct 1980

accgctgata agcagagggg agggaaaccc cctaccaagg cctga 2025

<210> 36
<211> 2025
<212> DNA
<213> Artificial Sequence

<220>
<223> an artificially synthesized sequence

<400> 36
atggccatcg gaggcaatta tgtgcatctg ccactctctg ccccaggatt tcaggccctg 60

tccgagggat gtacagccag ggacatcatc aacgaggagg ccgctgattg ggccatcgcc 120

ggcaccacat cttccgtgga tgaacaggcc cagctgggcc tgcagaagtg cgtgaggatg 180

tatgccaggt tttataaatc tctgagagct gagcaaaccg ctaaactggt gctgaaggga 240

ctgggcgtga acccagccat ggctgaggcc ctgaaggagg ctctggcacc tgccccactg 300

tctccccgca cactgaacgc ttgggtcgcc ggatgcaccc cttacgacat caaccaaatg 360

ctcgccgctg actgggatct gcagcatcca cagcctgctg ccgatgagca gatccagtgg 420

atgtaccgcc aacaggctag aatgtacaac cccacaaata tcctggatgt ggccgagcag 480

accgacgccg ctgtgaagaa ttggatggct gtgaatccaa cactggagga gatgctgaca 540

gctgctctgg ctcctgtgcc aatccccttc gccgcagccg ccgcctgggt gaagctgatc 600

gaggagaaaa aattcgccca gatgctgaat tgcgtggggg atcaccaagc cgctcagcca 660

gctcctcagc agggacagct gagagaggcc cgccagcaga acccaatccc cgtgggaaac 720

atcgccctgg acgtgaaaca gggccctaag gagcccttcg ctaactggat gacccagaca 780

ctgctgattc agaatgccct gaccgcttgt cagggagtgg gaggaccagg agccgccgct 840

gcccagcaga ggggaccaag gaagcctgct aaaaagttcg gagctgaggt ggtgccaggc 900

tttgcccacc aggccgctat gcagatcatc agggatatcg ctctgaggga gccaagcgga 960

tctgacatcg ctggcgccgg aaatatctat aggagatgga tccagctcgg cgctgagcca 1020

ttccagagct acgtggatag gttctatgcc atccagaacg ccaatcctga ctgcaaactc 1080

gtggctggac ctggacagaa agctcggctg atggccgagg ctaggaaacc tatcaagtgc 1140

tggaactgtg gaaaggccag gtggatcgcc gtgccaacct ggaggattcc cgccaaagtg 1200

tgctacgtgc cacatttcaa agtcggggcc caggatactt ggcatctgac ccctgagaag 1260

ggagctccca attacgctga tatcctgctg cactccaccg cttgctgtag gtttcctaga 1320

gcccataaat atcaggcacc aacctggaag cagtggcggc gcgacaacag ggccagaatc 1380

cccgagcggc tggagaggtg gcattccgcc aaagtggggt gggcttggtg gacatgttcc 1440

agagccgaga aaggatggct gagcacctac gccgtcagag ctcactctac ctatttccct 1500

tgctttacag ctggcgctaa ataccaggtg ccaagcctgc agtatctcgc tgctgacaat 1560

aggagaggac tgcgcatggc caagcaggct tggcactccc tgatcaagta cctgaagtac 1620

aaagcctgtt ctagagtgat ctttccactg caggaaggag ctgccgtgag aatcacatgg 1680

tattctaaga atttcgccac agccggcgag gtgaggagag ctatcagagg ggcctatctg 1740

gctctgaagg tggtgtccga tgtccgggcc gccaaacaga actccagggg cgataagcaa 1800

agggctaagt ataaaaccaa ggacctgcag aaagtctgcg cccaggaggg atcccacctg 1860

gaggtgcagg ggtacgccaa gaacttctgg accgacgtga cacccaacta cgctatcagg 1920

ggggagcagc tgctgtcctg ctgcagggct gatgtgcgga gccagggcga gaacccaaca 1980

tgggccaagc agaggggagg gaaaccccct acaaaaggcg cctga 2025

<210> 37
<211> 2025
<212> DNA
<213> Artificial Sequence

<220>
<223> an artificially synthesized sequence

<400> 37
atggccggag gcaattatgt gcatctgcca ctgtcccccg ccggatttca ggccctgtcc 60

gagggatgca cacctgccga catcatcaac gaggaggccg ctgattggga cgccgccggc 120

accacatctt ccgtggatga gcaaatcgcc ctgggcctgc agaagtgcgt gagaatgtat 180

aacgcctttt ataaatctct gagagctgag cagactgacg ctctggtgct gaagggactg 240

ggcgtgaatc ccacagccgc tgaggccctg aaggaggctc tggctccagt ggccctgtct 300

ccccgcacac tgaacgcctg ggtcaaggcc tgcacccctt acgacatcaa ccagatgctc 360

aatgccgact gggatctgca gcatccacag ccagcccctg ctgagcagat ccagtggatg 420

taccgccagc aaaacgctat gtacaacccc acaaatatcc tggacgtcaa agcccagacc 480

gacgccgctg tgaagaactg gatgactgcc aatccaacac tggaggagat gctgaccgcc 540

tgtgccgctc ctgtgccaat ccccttcgcc gctgctcagg cctgggtgaa gctgatcgag 600

gagaaaaagt ttggagccat gctgaattgc gtgggggatc accaggcagc tgccccagct 660

cctcagcagg gacagctgag ggaaccagcc cagcagaacc caatccccgt gggaaatatt 720

tatgccgacg tgaaacaggg ccctaaggag ccatttcagg cttggatgac ccagacactg 780

ctgatccaga atgccgccac cgcttgtcag ggagtgggag gacctggcca ggccgctgcc 840

cagcagaggg gaccaaggaa acccattgcc aagttcggag ctgaggtggt gccaggattc 900

caggctcagg ccgctatgca gatcatcagg gacattatcg ctagggagcc aagcggatct 960

gacatcgccg ggaccgccaa tatctatagg agatggatcc agctgggact ggccccattc 1020

cagagctacg tggataggtt ttacaaagct cagaacgcca atcctgactg caaactggtc 1080

ctggctcctg gacagaaagc tcggctgatg gctgaagccg ctaaacctat caagtgctgg 1140

aactgtggca aagaggcctg gatcgccgtg ccaacctgga gaatccctga ggctgtgtgc 1200

tacgtgccac atttcaaagt gggatgggcc ggatactggc atctgacccc tgagaaaggg 1260

tgggctaatt acgctgatat cctgctgcac tctacatatg cttgtaggtt tcctagagcc 1320

cataaatatc aggtcgccac ctggaagcag tggcggcgcg acaatagaag agccatcccc 1380

gagcggctgg agaggtggca cagcctggca gtggggtggg cttggtggac atgttctagg 1440

gtggccaaag gatggctgag cacctacgcc gtgcggatcg cctctaccta tttcccttgc 1500

tttacagccg ggaggcttta ccaggtgcca agcctgcagt atctggccct cgccaatagg 1560

agaggactgc gcatggccaa gcagaatgcc cactccctga tcaagtacct gaagtataag 1620

accgcttcta gagtgatctt tccactgcag gagggctccg ccgtgagaat cacatggtat 1680

tctaagaact tttgggctgc cggcgaggtg aggagagcta tcaggggaga ggcactggct 1740

ctgaaggtgg tgtccgatgt gaggagcgcc aaacagaact ccaggggcga taagcagaga 1800

ggagcctata aaaccaagga cctgcagaaa gtgtgttacg ccgagggatc ccacctggag 1860

gtgcagggat attgggccaa cttctggacc gacgtgacac ccaactacgc agccaggggg 1920

gagcagctgc tgtcctgctg tagatttgcc gtgcggagcc agggcgagaa cccaacatgg 1980

aaagctcaga ggggagggaa accccctaca aagggcgccg cctga 2025

<210> 38
<211> 2025
<212> DNA
<213> Artificial Sequence

<220>
<223> an artificially synthesized sequence

<400> 38
atggccggca attatgtgca tctgccactg tctcctcgcg cctttcaggc cctgtccgag 60

ggatgcaccc catacgccat catcaacgag gaggccgctg actgggacct ggccggcacc 120

acatcttccg tggatgagca gattcaggcc ggcctgcaga agtgcgtgag aatgtacaat 180

cccgcctata aatctctgag agctgagcag accgatgctg ccgtgctgaa gggactgggc 240

gtgaatccaa ccctggctga ggccctgaag gaggctctgg ctcctgtccc agcttctccc 300

cgcacactga acgcctgggt gaaactggcc accccttacg acatcaacca gatgctgaac 360

tgcgcctggg atctgcagca tccacagcca gctccccagg cccagatcca gtggatgtac 420

cgccagcaga atccagctta caaccccaca aatatcctgg acgtgaagca ggccaccgac 480

gccgctgtga agaactggat gacacaggct ccaacactgg aggagatgct gaccgcttgc 540

caagcccctg tgccaatccc cttcgccgct gcccaacagg cagtgaagct gatcgaggag 600

aaaaagttcg gcgctgccct gaattgcgtg ggggatcacc aggccgccat ggccgctcct 660

cagcagggac agctgaggga gcccagcgct cagaacccaa tccccgtggg aaatatctac 720

agggctgtga aacagggccc taaggagcca tttcagtccg ccatgaccca gacactgctg 780

atccagaacg ctaatgccgc ttgtcaggga gtgggaggac ctggccagaa ggccgcccag 840

cagaggggac caaggaaacc tattaaagcc ttcggagctg aggtggtgcc aggatttcaa 900

gccgctgccg ctatgcagat catcagggac atcattaacg ccgagccaag cggatctgac 960

atcgccggca ctacagccat ctataggaga tggatccagc tgggcctcca ggccttccag 1020

agctacgtgg ataggtttta taagtctgcc aacgccaatc ctgactgcaa actggtgctc 1080

aaggctggac agaaagctcg gctgatggct gaggctctcg ctcctatcaa gtgctggaac 1140

tgtggcaagg aaggggccat cgccgtgcca acctggagaa tccctgagag ggcctgctac 1200

gtgccacatt tcaaagtggg atgggccgcc tactggcatc tgacccctga gaaagggtgg 1260

ctcgcctacg ctgatatcct gctgcactct acctacttcg ccaggtttcc tagagcccat 1320

aaataccagg tcccagcctg gaagcagtgg cggcgcgaca ataggagggg agcccccgag 1380

cggctggaga ggtggcacag cctgattgcc gggtgggctt ggtggacatg ttctagagtc 1440

atcgccggat ggctgagcac ctacgccgtg agaattacag ctacctattt cccttgcttt 1500

acagccggcg aagtggccca ggtgccaagc ctgcagtatc tggctctcaa ggcaaggaga 1560

ggactgcgca tggccaaaca gaatagcgcc tccctgatca agtacctgaa gtataaaaca 1620

aaggccagag tgatctttcc actgcaggag ggaagccacg ccagaatcac atggtattct 1680

aagaactttt ggacagccgg cgaggtgagg agagctatca ggggggaaca agccgctctg 1740

aaggtggtgt ccgatgtgcg gtcccaggca cagaactcca ggggcgataa gcagaggggc 1800

ggggctaaaa ccaaggacct gcagaaagtg tgctatgtgg ctggatccca cctggaggtg 1860

cagggatatt ggcacgcttt ctggaccgac gtgacaccca attacgccga tgccggggag 1920

cagctgctgt cctgctgtag gttccctgcc cggagccagg gcgagaaccc aacctggaaa 1980

caggccaggg gagggaaacc ccctacaaag ggagctaacg cttga                    2025

<210>  39
<211>  42
<212>  DNA
<213>  Artificial Sequence

<220>
<223>  an artificially synthesized sequence

<400>  39
atatgcggcc gcgacgccac catggcctac cctgtgcagc ag                          42

<210>  40
<211>  68
<212>  DNA
<213>  Artificial Sequence

<220>
<223>  an artificially synthesized sequence

<400>  40
atatgcggcc gcgatgaact ttcaccctaa gtttttctta ctacggtcag gctttgcctc      60

ccctctgc                                                                68

<210>  41
<211>  43
<212>  DNA
<213>  Artificial Sequence

<220>
<223>  an artificially synthesized sequence

<400>  41
atatgcggcc gcgacgccac catggcccct gtgcagcaga tcg                         43

<210>  42
<211>  68
<212>  DNA
<213>  Artificial Sequence

<220>
<223>  an artificially synthesized sequence

<400>  42
atatgcggcc gcgatgaact ttcaccctaa gtttttctta ctacggtcag gcgggcttcc      60

ctcccctc                                                                68

<210>  43
<211>  43
<212>  DNA
<213>  Artificial Sequence

<220>
<223>  an artificially synthesized sequence

<400>  43
atatgcggcc gcgacgccac catggccgtg cagcagatcg gag                          43


<210>  44
<211>  69
<212>  DNA
<213>  Artificial Sequence

<220>
<223>  an artificially synthesized sequence

<400>  44
atatgcggcc gcgatgaact ttcaccctaa gtttttctta ctacggtcaa gcaggaggtt      60

tccctcccc                                                              69


<210>  45
<211>  42
<212>  DNA
<213>  Artificial Sequence

<220>
<223>  an artificially synthesized sequence

<400>  45
atatgcggcc gcgacgccac catggcccag cagatcggag gc                         42


<210>  46
<211>  69
<212>  DNA
<213>  Artificial Sequence

<220>
<223>  an artificially synthesized sequence

<400>  46
atatgcggcc gcgatgaact ttcaccctaa gtttttctta ctacggtcag gctgttgggg      60

gtttccctc                                                              69


<210>  47
<211>  46
<212>  DNA
<213>  Artificial Sequence

<220>
<223>  an artificially synthesized sequence

<400>  47
atatgcggcc gcgacgccac catggcccag atcggaggca attatg                     46


<210>  48
<211>  69
<212>  DNA
<213>  Artificial Sequence

<220>

<223>  an artificially synthesized sequence

<400>  48
atatgcggcc gcgatgaact ttcaccctaa gtttttctta ctacggtcag gccttggtag      60

ggggtttcc                                                              69


<210>  49
<211>  43
<212>  DNA
<213>  Artificial Sequence

<220>
<223>  an artificially synthesized sequence

<400>  49
atatgcggcc gcgacgccac catggccatc ggaggcaatt atg                        43


<210>  50
<211>  67
<212>  DNA
<213>  Artificial Sequence

<220>
<223>  an artificially synthesized sequence

<400>  50
atatgcggcc gcgatgaact ttcaccctaa gtttttctta ctacggtcag gcgccttttg      60

taggggg                                                                67


<210>  51
<211>  42
<212>  DNA
<213>  Artificial Sequence

<220>
<223>  an artificially synthesized sequence

<400>  51
atatgcggcc gcgacgccac catggccgga ggcaattatg tg                         42


<210>  52
<211>  69
<212>  DNA
<213>  Artificial Sequence

<220>
<223>  an artificially synthesized sequence

<400>  52
atatgcggcc gcgatgaact ttcaccctaa gtttttctta ctacggtcag gcggcgccct      60

ttgtagggg                                                              69


<210>  53
<211>  42
<212>  DNA

<213>  Artificial Sequence

<220>
<223>  an artificially synthesized sequence

<400>  53
atatgcggcc gcgacgccac catggccgga ggcaattatg tg                    42


<210>  54
<211>  69
<212>  DNA
<213>  Artificial Sequence

<220>
<223>  an artificially synthesized sequence

<400>  54
atatgcggcc gcgatgaact ttcaccctaa gtttttctta ctacggtcag gcggcgccct    60

ttgtagggg                                                            69

## Description

### [Technical Field]

The present invention relates to polypeptides that selectively induce antigen-specific CD8-positive T-cell responses while keeping antigen-specific CD4-positive T-cell responses at low levels, methods for producing such a polypeptide, vaccines expressing such a polypeptide, and the like. The vaccines of the present invention are particularly useful as anti-HIV vaccines.

### [Background Art]

The number of people infected with human immunodeficiency virus (HIV) exceeds 36 million worldwide, and around 1.8 million people are estimated to be newly infected annually. As such, the spread of HIV infection is a serious problem. The development of an HIV vaccine is an internationally important task to control the spread of HIV infection; however, no effective vaccine has yet been put into practical use. The HIV infection is a fatal infection which, in general, is not cured naturally and develops into chronic persistent infection, leading to acquired immunodeficiency syndrome (AIDS). Treatment with anti-HIV drugs has made it possible to prevent the onset of AIDS, but does not lead to cure because the virus is difficult to eliminate from the body. Therefore, infected persons need to be treated with anti-HIV drugs almost for life (NPL 12). Recently, in addition to the issues of side effects and emergence of drug-resistant virus under long-term medication, high medical costs and acceleration of disorders associated with chronic inflammation such as osteoporosis, cardiovascular disorders, brain and cognitive disorders, and renal disorders, have increasingly become serious problems (NPLs 13-15). However, the number of HIV-infected people is continuously rising in Africa and other parts of the world. In order to control the spread of infection, the development of an effective HIV vaccine is recognized as one of the most internationally important tasks, as well as the promotion of early diagnosis and early treatment. However, an HIV vaccine with established effectiveness has not been developed yet.

Induction of CD8-positive T-cell responses, which are believed to play a central role in suppressing HIV replication, is one of the key strategies in developing HIV vaccines (NPLs 1-4). In developing an HIV vaccine inducing CD8-positive T cells, optimization of antigen delivery and optimization of antigen are considered important. As for the delivery method, a number of vectors capable of efficiently inducing CD8-positive T cells, such as adenoviral vectors (NPL 7), cytomegalovirus vectors (NPL 8), and adenovirus/poxvirus vectors (NPL 9), have been developed.

On the other hand, antigens for inducing effective CD8-positive T cells may need further optimization. CD8-positive T cells specific for a viral antigen specifically recognize 8-to 11-mer peptide fragments (epitopes) derived from the viral antigen that are bound with the major histocompatibility complex (MHC) class I molecule and presented on the surface of virus-infected cells, and damage the infected cells (NPL 16). It is known that what is targeted by CD8-positive T-cell responses depends on the host's MHC class I genotype, and different target viral antigens cause the varying ability of CD8-positive T cells to suppress virus replication (effectiveness) (NPL 17). Moreover, domination of poorly effective CD8-positive T-cell responses results in inhibition of the induction of effective CD8-positive T-cell responses (immunodominance) (NPL 18). Therefore, an antigen needs to be designed so as to induce highly effective CD8-positive T-cell responses selectively. Recent analyses of HIV-infected individuals and simian AIDS models have shown that CD8-positive T-cell responses targeting Gag and Vif antigens have a strong ability to suppress viral replication (NPLs 17-20). The Gag capsid (CA) antigen is also promising as a candidate target region for CD8-positive T cells because of its highly conserved structure (NPL 21).

Conventional HIV vaccine methods induce not only HIV antigen-specific CD8-positive T cells but also HIV antigen-specific CD4-positive T cells at the same time. CD4-positive T cells specific for a viral antigen specifically recognize peptide fragments (epitopes) derived from the viral antigen that are bound with the MHC class II molecule and presented on the surface of antigen-presenting cells, and elicit antigen-specific responses. However, because HIV more preferentially targets HIV antigen-specific CD4-positive T cells to proliferate (NPL 10), vaccine-mediated induction of HIV antigen-specific CD4-positive T cells may lead to acceleration of HIV proliferation. In fact, the analysis of a simian immunodeficiency virus (SIV)-infected simian AIDS model reportedly showed that vaccine-mediated induction of SIV antigen-specific CD4-positive T cells was associated with acceleration of SIV proliferation in the acute phase after SIV exposure (NPL 11). Therefore, achieving antigen optimization requires not only designing a target of effective CD8-positive T cells but also developing a method for inducing effective HIV antigen-specific CD8-positive T cells selectively while suppressing the induction of HIV antigen-specific CD4-positive T cells as much as possible. However, antigen design from this point of view has not been done so far.

### [Citation List]

### [Non-Patent Literature]

[NPL 1] Koup RA. et al., J Virol. 68:4650-4655. 1994.
[NPL 2] Matano T. et al., J Virol. 72: 164-169. 1998.
[NPL 3] Schmitz JE. et al., Science. 283: 857-860. 1999.
[NPL 4] Goulder PJ. et al., Nat Rev Immunol. 4: 630-640. 2004.
[NPL 5] Matano T. et al., J Exp Med. 199: 1709-1718. 2004.
[NPL 6] Nyombayire J. et al., J Infect Dis. 215: 95-104. 2017.
[NPL 7] Wilson NA. et al., J Virol. 80: 5875-5885. 2006.
[NPL 8] Hansen SG et al., Nature. 473: 523-527. 2011.
[NPL 9] Barouch DH. et al., Nature. 482: 89-93. 2012.
[NPL 10] Douek DC. et al., Nature. 417: 95-98. 2002.
[NPL 11] Terahara K. et al., J Virol. 88: 14232-14240. 2014.
[NPL 12] Fischer M. et al., AIDS. 17: 195-199. 2003.
[NPL 13] Kirk GD. et al., Clin Infect Dis. 45: 103-110. 2007.
[NPL 14] Hsue PY. et al., Curr Opin HIV AIDS. 12: 534-539. 2017.
[NPL 15] Khoury G et al., J Infect Dis. 215: 911-919. 2017.
[NPL 16] Hewitt EW. et al., Immunology. 110: 163-169. 2003.
[NPL 17] Kiepiela P. et al., Nat Med. 13: 46-53. 2007.
[NPL 18] Akram A. et al., Clin Immunol. 143: 99-115. 2012.
[NPL 19] Mudd PA. et al., Nature. 491: 129-133. 2012.
[NPL 20] Iwamoto N. et al., J Virol. 88: 425-433. 2014.
[NPL 21] Goulder PJ. et al., Nat Rev Immunol. 8: 619-630. 2008.
[NPL 22] Tsukamoto T. et al., J Virol. 83: 9339-9346. 2009.
[NPL 23] Ishii H. et al., J Virol. 86:738-745. 2012.
[NPL 24] Letourneau S. et al., PLoS One. 2: e984. 2007.
[NPL 25] Mothe B. et al., J Transl Med. 9: 208. 2011.

### [Summary of Invention]

### [Technical Problem]

An objective of the present invention is to provide a polypeptide that selectively induces antigen-specific CD8-positive T cells while suppressing the induction of antigen-specific CD4-positive T cells, and a vaccine and the like containing that polypeptide.

### [Solution to Problem]

As stated above, the induction of CD8-positive T-cell responses, which are believed to play a central role in suppressing HIV replication, is one of the key strategies in developing HIV vaccines, and antigen design is considered important in developing a CD8-positive T cell-inducing HIV vaccine.

Thus, the present inventors contemplated designing an antigen based on a new method in order to selectively induce antigen-specific CD8-positive T cells while suppressing the induction of antigen-specific CD4-positive T cells. Specifically, first, the inventors divided the amino acid sequence of a target antigen protein into partial amino acid sequences having a length that is at least a length typical of MHC class I epitopes but not a length typical of MHC class II epitopes. The inventors then connected the divided partial amino acid sequences so as not to again form many consecutive partial amino acid sequences of the target antigen protein having a typical MHC class II epitope length, for example, by changing the order or placing overlaps and spacers. By doing so, the inventors formed an amino acid sequence containing MHC class I epitopes of the target antigen but not MHC class II epitopes of the target antigen.

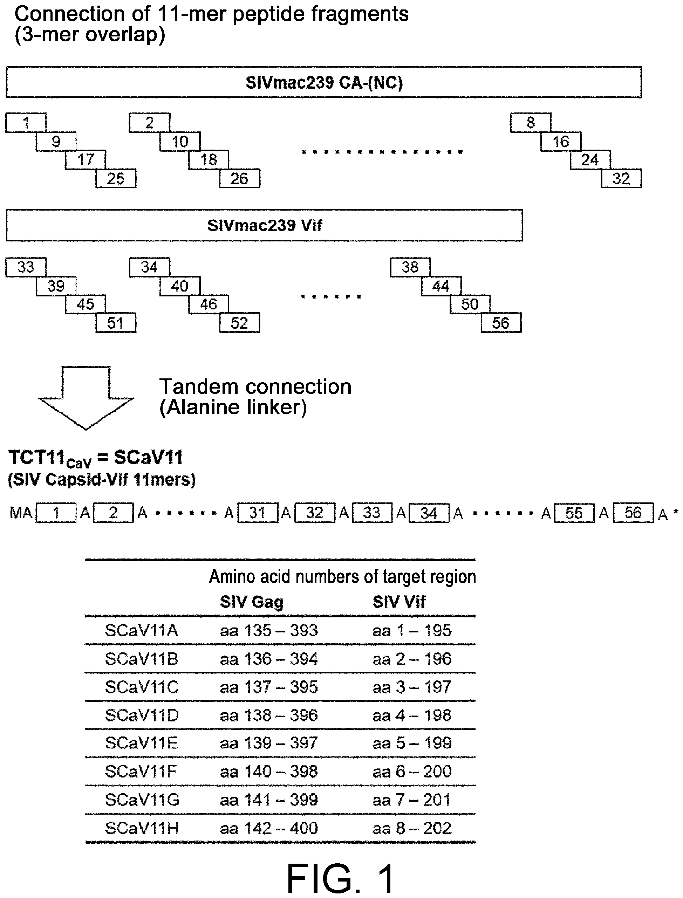

Specifically, in the Examples, noting the fact that optimum epitopes for CD4-positive T cells are 13- to 18-mer peptides while those for CD8-positive T cells are 8- to 11-mer peptides, the present inventors designed novel antigens by connecting 11-mer peptides derived from HIV target antigens in tandem in order to induce effective HIV antigen-specific CD8-positive T cells selectively. The antigens were designed by fragmenting the amino acid sequences of viral Gag CA and Vif, which are target regions for effective CD8-positive T cells, into 11-mer peptides, then rearranging these peptides and connecting them in tandem using alanine as spacers (TCT11) (Fig. 1). In a similar manner, a total of 8 tandemly-connected antigens (A to H) were designed, for each of which the starting amino acid position of the peptides in each target antigen region was shifted by one amino acid. All 8 antigens cover all theoretically possible CD8-positive T-cell epitopes present in the target regions. Meanwhile, these antigens do not contain virus-derived 12-mer or longer peptides, and therefore, theoretically, do not contain optimum epitopes for viral antigen-specific CD4-positive T cells. Thus, they should not efficiently induce CD4-positive T cells.

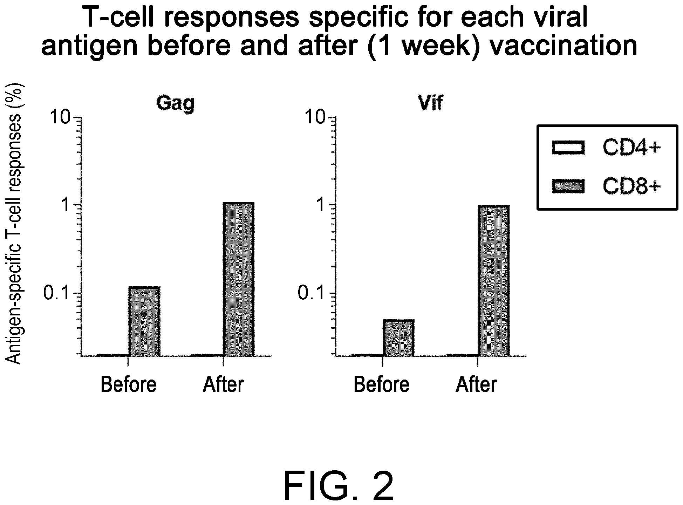

Although the thus designed tandemly-connected antigens correspond to the amino acid sequences of the target antigens in the short range of 11-mer, their whole amino acid sequences are completely different from those of the target antigens. To examine whether these connected antigens can efficiently induce specific CD8-positive T cells against the target antigen proteins, viral vectors expressing the connected antigens were constructed and inoculated into individuals. As a result, the inoculated individuals were found to show a significantly increased frequency of target antigen-specific CD8-positive T cells, but no change in the frequency of target antigen-specific CD4-positive T cells (Fig. 2). Thus, it was demonstrated that a polypeptide produced by rearranging the amino acid sequence of an antigen protein according to the present invention can be used as an antigen to selectively induce immune responses mediated by MHC class I epitopes of the target antigen.

With the method of the present invention, it is not necessary to identify an MHC class I epitope of a target antigen in advance. A polypeptide constructed by rearranging the amino acid sequence according to the method of the present invention can be inoculated as an antigen to selectively induce immune responses mediated by MHC class I epitopes of the target antigen while avoiding the induction of immune responses mediated by MHC class II epitopes of the target antigen. This method is highly versatile and can be used not only for infectious viruses such as HIV but for a wide range of target proteins in general to selectively induce the response of target antigen-specific CD8-positive T cells.

Thus, the present invention relates to antigens that selectively induce antigen-specific CD8-positive T cells while suppressing the induction of antigen-specific CD4-positive T cells, and vaccines and the like including such an antigen. More specifically, the present invention relates to each of the inventions recited in the claims. It should be noted that inventions consisting of any combination of two or more inventions recited in claims that refer to the same claim are also intended herein. Specifically, the present invention relates to the following:
[1] A polypeptide comprising multiple peptides connected together, wherein each of the multiple peptides has an amino acid sequence of eight to twelve residues included in the amino acid sequence of an antigen protein.
[2] The polypeptide of [1], wherein the eight- to twelve-residue peptides are connected in an order different from that in the antigen protein.
[3] The polypeptide of [1] or [2], which does not substantially comprise a partial amino acid sequence of 13 or more consecutive residues in the antigen protein.
[4] The polypeptide of any one of [1] to [3], wherein the amino acid sequences of the multiple peptides optionally comprise an overlap.
[5] The polypeptide of [4], wherein the overlap consists of one to four residues.
[6] The polypeptide of any one of [1] to [5], wherein each of the connection sites optionally comprises a spacer.
[7] The polypeptide of [6], wherein the spacer consists of one to four amino acid residues.
[8] The polypeptide of any one of [1] to [7], wherein at least 20 eight- to twelve-residue peptides are connected together.
[9] A nucleic acid encoding the polypeptide of any one of [1] to [8].
[10] A vector comprising the nucleic acid of [9].
[11] The vector of [10], which is a Sendai virus vector.
[12] A vaccine comprising the polypeptide of any one of [1] to [8], a nucleic acid encoding the polypeptide, or a vector comprising the nucleic acid.
[13] The vaccine of [12], wherein the antigen protein is derived from an antigen protein of a human immunodeficiency virus.
[14] A method for selectively inducing CD8-positive T cells specific for a target antigen, which comprises inoculating the vaccine of [12] or [13].
[15] A method for producing the polypeptide of [1] or a nucleic acid encoding the polypeptide, which comprises:
   (i) dividing an amino acid sequence encoding an antigen protein into amino acid sequences of eight to twelve residues, wherein the divided amino acid sequences may or may not overlap with one another;
   (ii) connecting the divided amino acid sequences in such a way as not to become the same as the amino acid sequence of the antigen protein, wherein a spacer may or may not be inserted in each of the connection sites of the divided amino acid sequences; and
   (iii) obtaining a polypeptide comprising an amino acid sequence resulting from step (ii) or a nucleic acid encoding the polypeptide.

   In addition, the present invention also encompasses the following inventions:
[16] The polypeptide of any one of [1] to [8], wherein the multiple peptides are connected together *via* a spacer.
[17] The polypeptide of any one of [1] to [8] and [16], wherein 10 or more eight- to twelve-residue amino acid sequences are connected together.
[18] The polypeptide of [17], wherein 20 or more eight- to twelve-residue amino acid sequences are connected together.
[19] The polypeptide of [17], wherein 30 or more 8- to 12-residue amino acid sequences are connected together.
[20] The polypeptide of any one of [1] to [8] and [16] to [19], wherein the amino acid sequence of 8 to 12 residues or less is an amino acid sequence of 8 to 11 residues.
[21] The polypeptide of any one of [1] to [8] and [16] to [20], wherein the total number of residues of a partial amino sequence of consecutive 13 or more residues of the antigen protein is 20% or less of the total number of residues of the connected amino acid sequences.
[22] The polypeptide of [21], wherein the total number of residues of a partial amino sequence of consecutive 13 or more residues of the antigen protein is 10% or less or 5% or less of the total number of residues of the connected amino acid sequences.
   Furthermore, the present invention also encompasses the following inventions:
[23] The polypeptide of any one of [1] to [8], which comprises an amino acid sequence in which multiple amino acid sequences of 12 residues or less selected from the amino acid sequence of the antigen protein are connected together.
[24] The polypeptide of [23], wherein the multiple amino acid sequences are connected so as not to become the same amino acid sequence of the antigen protein.
[25] The polypeptide of [23] or [24], wherein the multiple amino acid sequences are connected so as not to substantially generate an amino acid sequence of consecutive 13 or more residues of the antigen protein.
[26] The polypeptide of [23] or [24], wherein the multiple amino acid sequences are connected such that the number of connections generating an amino acid sequence of consecutive 13 or more residues of the antigen protein is 20% or less, 10% or less, or 5% or less of the total number of connections.
[27] The polypeptide of [26], wherein multiple amino acid sequences of 11 residues or less selected from the amino acid sequence of the antigen protein are connected together, wherein the multiple amino acid sequences are connected so as not to substantially generate an amino acid sequence of consecutive 12 or more residues of the antigen protein, or such that the number of connections generating an amino acid sequence of consecutive 12 or more residues of the antigen protein is 20% or less, 10% or less, or 5% or less of the total number of connections.
[28] The polypeptide of any one of [23] to [27], wherein the multiple peptides are connected together *via* a spacer.
[29] The polypeptide of [28], wherein the spacer consists of 1 to 4 amino acid residues.
[30] The polypeptide of any one of [20] to [29], wherein the divided amino acid sequences overlap one another.
[31] The polypeptide of [30], wherein the divided amino acid sequences overlap by 1 to 4 residues.
[32] The polypeptide of any one of [23] to [31], wherein at least 10 or more divided amino acid sequences are connected together.
[33] The polypeptide of [32], wherein 20 or more divided amino acid sequences are connected together.
[34] The polypeptide of [32], wherein 30 or more divided amino acid sequences are connected together.
[35] The polypeptide of any one of [23] to [34], wherein the amino acid sequence of the antigen protein is divided into amino acid sequences of 5 to 12 residues.
[36] The polypeptide of any one of [23] to [34], wherein the amino acid sequence of the antigen protein is divided into amino acid sequences of 8 to 12 residues.
[37] The polypeptide of any one of [23] to [34], wherein the amino acid sequence of the antigen protein is divided into amino acid sequences of 8 to 11 residues.
   Furthermore, the present invention also encompasses the following inventions:
[38] A nucleic acid encoding the polypeptide of any one of [16] to [37].
[39] A vector comprising the nucleic acid of [38].
[40] The vector of [39], which is a Sendai virus vector.
[41] A vaccine comprising the polypeptide of any one of [16] to [37], a nucleic acid encoding the polypeptide, or a vector comprising the nucleic acid.
[42] The vaccine of [41], wherein the antigen protein is derived from an antigen protein of a human immunodeficiency virus.
[43] A method for selectively inducing CD8-positive T cells specific for a target antigen, which comprises inoculating the vaccine of [41] or [42].
   Furthermore, the present invention also encompasses the following inventions:
[44] Use of the polypeptide of any one of [1] to [8] and [16] to [37], a nucleic acid encoding the polypeptide, or a vector comprising the nucleic acid, for selectively inducing target antigen-specific CD8-positive T cells.
[45] Use of the polypeptide of any one of [1] to [8] and [16] to [37], a nucleic acid encoding the polypeptide, or a vector comprising the nucleic acid, for manufacture of a medicament or an agent for selectively inducing target antigen-specific CD8-positive T cells.
   Furthermore, the present invention also encompasses the following inventions:
[46] A method of vaccination comprising inoculating the polypeptide of any one of [1] to [8] and [16] to [37], a nucleic acid encoding the polypeptide, or a vector comprising the nucleic acid.
[47] The method of [46], wherein a plurality of the polypeptides of any one of [1] to [8] and [16] to [37], a plurality of nucleic acids encoding the polypeptides, or a plurality of vectors comprising the nucleic acids, are administered.
[48] The method of [46] or [47], which comprises further inoculating an additional polypeptide that is not the polypeptide of any one of [1] to [8] and [16] to [37], a nucleic acid encoding the additional polypeptide, or a vector comprising the nucleic acid.
[49] The method of [48], wherein the additional polypeptide that is not the polypeptide of any one of [1] to [8] and [16] to [37], the nucleic acid encoding the polypeptide, or the vector comprising the nucleic acid, is inoculated first.
[50] The method of [48] or [49], wherein the additional polypeptide is the antigen protein or a partial peptide thereof.
[51] The method of any one of [48] to [50], wherein the vector comprising the nucleic acid encoding the additional polypeptide is a DNA vector.
[52] Use of the polypeptide of any one of [1] to [8] and [16] to [37], a nucleic acid encoding the polypeptide, or a vector comprising the nucleic acid, for vaccination.
[53] The use of [52], which is for administering a plurality of the polypeptides of any one of [1] to [8] and [16] to [37], a plurality of nucleic acids encoding the polypeptides, or a plurality of vectors comprising the nucleic acids.
[54] The use of [52] or [53], wherein an additional polypeptide that is not the polypeptide of any one of [1] to [8] and [16] to [37], a nucleic acid encoding the additional polypeptide, or a vector comprising the nucleic acid, is further inoculated.
[55] The method of [54], wherein the additional polypeptide that is not the polypeptide of any one of [1] to [8] and [16] to [37], the nucleic acid encoding the polypeptide, or the vector comprising the nucleic acid, is inoculated first.
[56] The use of [54] or [55], wherein the additional polypeptide is the antigen protein or a partial peptide thereof.
[57] The method of any one of [54] to [56], wherein the vector comprising the nucleic acid encoding the additional polypeptide is a DNA vector.
[58] Use of the polypeptide of any one of [1] to [8] and [16] to [37], a nucleic acid encoding the polypeptide, or a vector comprising the nucleic acid, for manufacture of a medicament or an agent for vaccination.
[59] The use of [58], which is for administering a plurality of the polypeptides of any one of [1] to [8] and [16] to [37], a plurality of nucleic acids encoding the polypeptides, or a plurality of vectors comprising the nucleic acids.
[60] The use of [58] or [59], wherein an additional polypeptide that is not the polypeptide of any one of [1] to [8] and [16] to [37], a nucleic acid encoding the additional polypeptide, or a vector comprising the nucleic acid, is further inoculated.
[61] The method of [60], wherein the additional polypeptide that is not the polypeptide of any one of [1] to [8] and [16] to [37], the nucleic acid encoding the polypeptide, or the vector comprising the nucleic acid, is inoculated first.
[62] The use of [60] or [61], wherein the additional polypeptide is the antigen protein or a partial peptide thereof.
[63] The method of any one of [60] to [62], wherein the vector comprising the nucleic acid encoding the additional polypeptide is a DNA vector.
[64] Use of the polypeptide of any one of [1] to [8] and [16] to [37], a nucleic acid encoding the polypeptide, or a vector comprising the nucleic acid, for vaccination.
[65] The use of [64], which is for administering a plurality of the polypeptides of any one of [1] to [8] and [16] to [37], a plurality of nucleic acids encoding the polypeptides, or a plurality of vectors comprising the nucleic acids.
[66] The use of [64] or [65], wherein an additional polypeptide that is not the polypeptide of any one of [1] to [8] and [16] to [37], a nucleic acid encoding the additional polypeptide, or a vector comprising the nucleic acid, is further inoculated.
[67] The method of [66], wherein the additional polypeptide that is not the polypeptide of any one of [1] to [8] and [16] to [37], the nucleic acid encoding the polypeptide, or the vector comprising the nucleic acid, is inoculated first.
[68] The use of [66] or [67], wherein the additional polypeptide is the antigen protein or a partial peptide thereof.
[69] The method of any one of [66] to [68], wherein the vector comprising the nucleic acid encoding the additional polypeptide is a DNA vector.

It should be noted that any technical matter or any combination of technical matters described in the present specification are intended herein. In addition, inventions that correspond to those inventions except that any matter or any combination of matters described in the present specification are excluded are also intended herein. Moreover, a specific embodiment described herein in relation to the present invention is meant to disclose not only that embodiment but also an invention corresponding to a more generic invention disclosed herein including that embodiment from which that embodiment is excluded.

### Effects of the Invention

As stated above, the present invention is useful for selectively inducing CD8-positive T cells for a desired antigen. For example, the present invention enables an AIDS vaccine to induce effective HIV antigen-specific CD8-positive T cells selectively while suppressing the induction of HIV antigen-specific CD4-positive T cells as much as possible.

### [Brief Description of Drawings]

Fig. 1 shows a summary of target 11-mer connected antigen TCT11.
Fig. 2 shows an inoculation test of SCaV11 antigen-expressing vaccines in the chronic phase of SIV replication-controlled monkeys.
Fig. 3 shows an inoculation test of SCaV11 antigen-expressing vaccines in non-infected monkeys.
Fig. 4 shows antigen-specific T-cell responses after vaccination as in Fig. 3.
Fig. 5 shows antigen-specific T-cell responses in the lymph node after vaccination.

### [Description of Embodiments]

The embodiments of the present invention are specifically described below.

In the present invention, a "vaccine" refers to a composition for eliciting immune responses against an antigen. For example, it refers to a composition used for preventing or treating a contagious disease, an infection, and the like. A vaccine contains an antigen or can express an antigen, by which it can induce immune responses against the antigen. The polypeptides of the present invention, and nucleic acids and vectors encoding the polypeptides, are useful as vaccines for preventing or treating the infection, transmission, and epidemic of pathogenic microorganisms. The vaccines can be used in any form as desired.

An "antigen" refers to, in general, a molecule that contains one or more epitopes (portions of antigen recognized by antibodies or immune cells) and may stimulate the host immune system and induce antigen-specific immune responses. The immune responses may be humoral immune responses and/or cellular immune responses. In the present invention, the epitopes include not only epitopes formed from primary structures but also epitopes depending on protein conformations. The "antigen" is also referred to as "immunogen".

In the present invention, a "viral vector" is a vector having genomic nucleic acid derived from a virus, and capable of expressing a transgene incorporated into the nucleic acid after being introduced into cells. For example, paramyxovirus vectors are chromosomally non-integrating viral vectors and expressed within the cytosol. Therefore, they have no risk of integrating a transgene into host chromosomes (nuclear chromosomes). They are therefore highly safe, and can also be removed from infected cells. In the present invention, paramyxovirus vectors include infectious viral particles, and also include viral cores, complexes composed of a viral genome and viral proteins or complexes composed of a non-infectious viral particle and such that are capable of expressing a gene they carry when introduced into cells. For example, in the paramyxovirus, the ribonucleoprotein (viral core) composed of a paramyxovirus genome and paramyxovirus proteins binding to it (NP, P, and L proteins) can express a transgene intracellularly when introduced into cells (WO00/70055). The introduction into cells may be performed using a transfection agent and such, as appropriate. Such ribonucleoproteins (RNPs) are also included in paramyxovirus vectors in the present invention. Preferably, a paramyxovirus vector in the present invention is a particle in which the aforementioned RNP is enclosed by a biological membrane derived from the cell membrane.

The present invention provides polypeptides useful for selectively inducing CD8-positive T cells specific for a target antigen protein while suppressing the induction of CD4-positive T cells specific for the target. Such a polypeptide contains an amino acid sequence in which partial amino acid sequences excised from an antigen protein such that they have a length that is at least a length typical of MHC class I epitopes but not a length typical of MHC class II epitopes are connected together in such a way as not to become the same as the original amino acid sequence of the antigen protein *(i.e.* the amino acid sequence of the antigen protein). Here, the length typical of MHC class I or MHC class II epitopes refers to a typical length required for MHC class I or MHC class II epitopes. MHC class I epitopes generally have about 5 to 12 residues. Optimum MHC class I epitopes for inducing CD8-positive T cells are 8- to 11-residue peptides. On the other hand, optimum MHC class II epitopes for inducing CD4-positive T cells are considered to be 13- to 18-residue peptides. In the present invention, the length typical of MHC class I epitopes is, for example, 5 to 12 amino acids, preferably 6 to 12 amino acids, more preferably 7 to 12 amino acids, still more preferably 8 to 11 amino acids, and for example, 7, 8, 9, 10, or 11 amino acids. In the present invention, the length typical of MHC class II epitopes is, for example, 15 amino acids or longer, preferably 14 to 25 amino acids or longer, more preferably 13 to 18 amino acids, and for example, 22, 20, 18, 15, or 13 amino acids.

A polypeptide of the present invention can be produced, for example, by the following steps:
(i) dividing an amino acid sequence encoding a desired target antigen protein into amino acid sequences having a length that is at least a length typical of MHC class I epitopes but not a length typical of MHC class II epitopes, wherein the divided amino acid sequences may or may not overlap with one another;
(ii) connecting the divided amino acid sequences in such a way as not to become the same as the amino acid sequence of the antigen protein *(i.e.* the original amino acid sequence), wherein a spacer may or may not be inserted in each of the connection sites of the divided amino acid sequences; and
(iii) obtaining a polypeptide comprising an amino acid sequence resulting from step (ii).

In addition, a nucleic acid encoding a polypeptide of the present invention can be produced, for example, by the following steps:
(i) dividing an amino acid sequence encoding a desired target antigen protein into amino acid sequences having a length that is at least a length typical of MHC class I epitopes but not a length typical of MHC class II epitopes, wherein the divided amino acid sequences may or may not overlap with one another;
(ii) connecting the divided amino acid sequences in such a way as not to become the same as the amino acid sequence of the antigen protein *(i.e.* the original amino acid sequence), wherein a spacer may or may not be inserted in each of the connection sites of the divided amino acid sequences; and
(iii) obtaining a nucleic acid encoding a polypeptide comprising an amino acid sequence resulting from step (ii).

Here, the "length that is at least a length typical of MHC class I epitopes but not a length typical of MHC class II epitopes" refers to, for example, a length of 5 to 14 amino acids, preferably 5 to 13 amino acids, more preferably 5 to 12 amino acids, still more preferably 8 to 12 amino acids, still more preferably 8 to 11 amino acids.

A target antigen protein is not particularly limited, and may be any desired protein. An antigen protein may be a natural protein or an artificial protein, but preferably is a natural protein. A full-length protein or a partial protein thereof may be used as an antigen protein. A fusion protein in which multiple proteins are linked together may also be used as an antigen protein. An antigen protein used in the present invention is preferably a protein associated with a disease. Particularly preferred is an antigen protein against which the induction of cellular immunity leads to prevention and/or treatment of a disease. Typical target antigen proteins include a protein of a desired pathogen, pathogenic microorganism, parasite, or such, or a fragment thereof; and a cancer antigen (tumor-specific protein) or cancer stem cell antigen, or a fragment thereof. Examples of tumor antigens include, for example, WT1, survivin, survivin-B2, MAGE-A3, MEGE-A4, tyrosinase, gp100, Melan-A, TRP-2, SNRPD1, CDK4, NY-ESO-1, HER2, MUC-1, CD20, and p53. Cancer stem cell antigens include CD44, CD133, LGR5, and Dclk1. Viral antigens include component proteins of viruses such as hepatitis virus (such as HBV and HCV), human papilloma virus, human immunodeficiency virus, and adult T-cell leukemia virus. Parasite antigens include *Plasmodium* proteins.

Antigen proteins include, in particular, proteins derived from infectious microorganisms, particularly pathogenic viruses, more specifically CD4-positive T cell-infecting viruses. Such viruses include human immunodeficiency virus (HIV), which causes acquired immunodeficiency syndrome (AIDS), and human T-cell leukemia virus (HTLV-1), which causes adult T-cell leukemia (ATL). A protein of these viruses or a fragment thereof can be suitably used as an antigen protein of the present invention.

The present invention also relates to polypeptides comprising multiple peptides connected together, wherein each of the multiple peptides has an amino acid sequence of 12 residues or less included in the amino acid sequence of a desired antigen protein. The term "12 residues or less" is not particularly limited in its lower limit as long as the amino acid sequence has such a length as to be a potential MHC class I epitope, and refers to, for example, 5 to 12 amino acids, preferably 6 to 12 amino acids, more preferably 7 to 12 amino acids, still more preferably 8 to 12 amino acids, 9 to 12 amino acids, 10 to 12 amino acids, or 10 to 11 amino acids. The term "multiple" may be any plural number as long as the peptides are expected to actually include a peptide serving as an MHC class I epitope, and refers to, for example, 10 or more, preferably 15 or more, more preferably 20 or more, for example, 30 or more, 40 or more, or 50 or more.

Specifically, a polypeptide of the present invention may contain an amino acid sequence in which partial amino acid sequences (also referred to as divided amino acid sequences) excised from the amino acid sequence of a desired antigen protein such that they have at least 5 consecutive amino acids thereof (preferably 6, 7, 8, 9, 10, or 11 consecutive amino acids thereof), but not 15 consecutive amino acids thereof (preferably 14, 13, or 12 consecutive amino acids thereof), are connected in such a way as not to become the same as the original amino acid sequence of the antigen protein. More specifically, a polypeptide of the present invention may contain an amino acid sequence in which partial amino acid sequences excised from the amino acid sequence of a desired antigen protein such that they have at least 8 amino acids thereof (more preferably at least 9, 10, or 11 amino acids thereof), but not 13 amino acids thereof (more preferably 12 amino acids thereof), are connected in such a way as not to become the same as the original amino acid sequence of the antigen protein.

The phrase "the same as the original amino acid sequence of the antigen protein" means that the amino acid sequence finally generated by connection is identical to the amino acid sequence of the antigen protein (the amino acid sequence of the antigen protein before division). In order not to become the same as the original amino acid sequence, for example, the partial sequences are connected in an altered order, for example, in a non-consecutive order, in a random order, or in no particular order. Alternatively, the partial sequences can be connected *via* an intervening spacer consisting of one or more amino acids so that even an amino acid sequence generated by connecting them sequentially will not be the same as the original amino acid sequence. Alternatively, if partial sequences are divided from the amino acid sequence of an antigen protein such that they have an overlap of several residues, an amino acid sequence generated by connecting them will not be the same as the original amino acid sequence of the antigen protein.

The manner of excising partial amino acid sequences from the amino acid sequence of an antigen protein is not particularly limited. Sequence fragments may be excised such that all fragments have the same length (for example, a common length of 8, 9, 10, 11, or 12 amino acids) or different fragments have different lengths (for example, each fragment is 8, 9, 10, 11, or 12 amino acids long), but typically in the former manner.

For example, cases of dividing the following antigen protein amino acid sequence are exemplified below:

YPVQQIGGNYVHLPLSPRTLNAWVKLIEEKKFGAEVVPGFQALSEGCTPYDINQMLNCV
G... (SEQ ID NO: 1).

When this sequence is divided into, for example, 11-residue amino acid sequences, for example, it can be divided as follows:

### [Case 1]

YPVQQIGGNYV (SEQ ID NO: 2)
HLPLSPRTLNA (SEQ ID NO: 3)
WVKLIEEKKFG (SEQ ID NO: 4)
AEVVPGFQALS (SEQ ID NO: 5)
EGCTPYDINQM (SEQ ID NO: 6)

The manner of division shown above allows the entire *(i.e.* 100%) amino acid sequence of the antigen protein to be divided into 11-residue amino acid sequences except for the last remaining portion of less than 11 residues (if such a portion occurs, though). In the present invention, this is called a ratio of coverage of the amino acid sequence of the antigen protein. In the above case, it is almost 100%. A connected amino acid sequence included in a polypeptide of the present application has, for example, 50% or higher, preferably 55% or higher, 60% or higher, 65% or higher, 70% or higher, 75% or higher, 80% or higher, 85% or higher, 90% or higher, 95% or higher, or 100% coverage of the amino acid sequence of an antigen protein.

If these divided amino acid sequences (in the above case, SEQ ID NOs: 2-6) are connected sequentially, the connected sequence will be back to the original amino acid sequence of the antigen protein (or in other words, become the same as the original amino acid sequence). To avoid this, the order of connection can be changed appropriately. For example, SEQ ID NOs: 2, 4, 3, 5, ... can be connected in this order so that the connected sequence will not become the same as the original amino acid sequence. Such a manner of connection is not particularly limited. The divided sequences may be connected in a non-consecutive but consistent order, or at random. Random connection may probabilistically result in fragments originally next to each other being again connected next to each other. Such connections are acceptable to some extent, but preferably should be avoided as much as possible. For example, in a connected amino acid sequence included in a polypeptide of the present invention, the number of connections that result in fragments originally next to each other being again connected next to each other and thereby yield a connected amino acid sequence portion identical to the corresponding original amino acid sequence of the antigen protein is, for example, 10% or less, preferably 8% or less, more preferably 5% or less, even more preferably 3% or less, and still more preferably 1% or less, of the total number of connections included in the connected amino acid sequence. Obviously, it is most preferable not to include such connections.

For example, it is preferred that a connected amino acid sequence does not substantially contain a partial amino acid sequence of longer than 15 consecutive amino acids (preferably, at least longer than 14, 13, 12, or 11 amino acids) of the original amino acid sequence of the antigen protein. The term "not substantially contain" means that the connected amino acid sequence does not contain such a long consecutive partial amino acid sequence, or that the total number of residues of such a long consecutive partial amino acid sequence is sufficiently smaller than the total number of residues of the connected amino acid sequence. "Sufficiently smaller" means, for example, that the total number of residues of such a long consecutive partial amino acid sequence is preferably 30% or smaller, more preferably 25% or smaller, still more preferably 20% or smaller, even more preferably 15% or smaller, still more preferably 10% or smaller, or even more preferably 5% or smaller, of the total number of residues of the connected amino acid sequence. For example, the connected amino acid sequence does not contain a partial amino acid sequence of longer than 12 consecutive amino acids (preferably longer than 11 amino acids) of the original antigen protein, or alternatively, the total number of residues of such a partial amino acid sequence is 10% or smaller (more preferably 5% or smaller) of the total number of residues of the connected amino acid sequence.

Divided amino acid sequences may be connected *via* a spacer. A spacer may consist of one or more amino acid residues, preferably one to several amino acid residues, for example, 1, 2, 3, or 4 desired amino acid residues. Amino acid to be used as a spacer is not particularly limited. For example, alanine (A) can be used. For example, the divided amino acid sequences exemplified above (SEQ ID NOs: 2-6) can be connected *via* a spacer so that even a sequence generated by connecting them sequentially will not be back to the original amino acid sequence of the antigen protein (or in other words, not become the same as the original amino acid sequence). Of course, a spacer may also be appropriately inserted when connecting divided amino acid sequences non-consecutively or randomly.

The manner of dividing the amino acid sequence of an antigen protein is not limited to the one mentioned above. For example, 8- to 12-residue amino acid sequences may be excised from anywhere in the amino acid sequence of an antigen protein. For example, when the antigen protein amino acid sequence shown above (SEQ ID NO: 1) is divided into 11-residue amino acid sequences separated with a gap of 3 residues, it can be divided as follows:

### [Case 2]

YPVQQIGGNYV (SEQ ID NO: 2)
LSPRTLNAWVK (SEQ ID NO: 7)
EKKFGAEVVPG (SEQ ID NO: 8)
LSEGCTPYDIN (SEQ ID NO: 9)

In this case, the ratio of coverage of the amino acid sequence of the antigen protein is 11/14, *i.e.* 78.6%, provided that the last remaining portion of less than 11 residues is excluded. These divided amino acid sequences can be connected in any desired order. For example, they can be connected in the same order as original, in a non-consecutive order, or in a random order. A spacer may or may not be inserted in connection sites.

Amino acid sequences divided from the amino acid sequence of an antigen protein may overlap with one another. For example, dividing the antigen protein amino acid sequence shown above (SEQ ID NO: 1) into 11-residue amino acid sequences with an overlap of 3 residues with one another will result in the following sequences:

### [Case 3]

YPVQQIGGNYV (SEQ ID NO: 2)
NYVHLPLSPRT (SEQ ID NO: 10)
PRTLNAWVKLI (SEQ ID NO: 11)
KLIEEKKFGAE (SEQ ID NO: 12)
GAEVVPGFQAL (SEQ ID NO: 13)
QALSEGCTPYD (SEQ ID NO: 14)
PYDINQMLNCV (SEQ ID NO: 15)

Making overlaps as shown above is advantageous in that a wider variety of divided sequences having such a length as to be potential MHC class I epitopes can be incorporated into a connected amino acid sequence. For example, in the case of dividing the amino acid sequence of an antigen protein into 11-amino acid fragments without gaps or overlaps as in "Case 1" and connecting them to produce a polypeptide, the amino acid sequence may be divided in 11 different frames. Specifically, "Case 1" above shows the case where the antigen protein amino acid sequence is divided into 11-amino acid fragments starting from the 1st amino acid. In addition to this, the amino acid sequence may be divided into 11-amino acid fragments starting from the 2nd amino acid, the 3rd amino acid, ..., and the 11th amino acid. Therefore, in order to cover all 11-amino acid divided sequences in all frames, 11 connected amino acid sequences are required. However, in the case where amino acid sequences divided such that they have an overlap of 3 residues are connected to produce a connected amino acid sequence as in "Case 3", only 8 connected amino acid sequences are required to cover all 11-amino acid divided sequences in all frames (see the Examples). Thus, by providing an overlap between divided amino acid sequences, all theoretically possible divided amino acid sequences (that is, potential MHC class I epitope sequences) present in the amino acid sequence of the antigen protein can be covered using fewer connected amino acid sequences.

When an overlap is provided, the length of the overlap is not particularly limited. However, when the connected polypeptide is expressed as a recombinant protein and such, the length of overlapping regions should preferably not be very long in order to avoid unwanted events caused by homologous recombination such as sequence deletion and duplication. The length of an overlap between divisional amino acid sequences is, for example, one to several residues, and specifically, for example, 1 to 6 amino acids, more preferably 1 to 5 amino acids, even more preferably 1 to 4 amino acids, still more preferably 1 to 3 amino acids, and even more preferably 1 to 2 amino acids.

In the cases shown above, the amino acid sequence of the antigen protein is divided into sequences of a fixed number of amino acids (in the above cases, 11 amino acids). However, the number of amino acids does not need to be fixed. For example, in the case below, the antigen protein amino acid sequence of SEQ ID NO: 1 is divided into sequences of 11 amino acids, 8 amino acids, 10 amino acids, 9 amino acids, and 11 amino acids in this order. Each divided amino acid sequence may or may not have a gap, and may or may not have an overlap. The present invention also encompasses embodiments where such divided amino acid sequences are connected.

### [Case 4]

YPVQQIGGNYV (SEQ ID NO: 2)
NYVHLPLS (SEQ ID NO: 16)
SPRTLNAWVK (SEQ ID NO: 17)
EEKKFGAEV (SEQ ID NO: 18)
EVVPGFQALSE (SEQ ID NO: 19)

Typically, the amino acid sequence of an antigen protein is divided into sequences of any fixed number of amino acids selected from 8 to 12 (for example, if " 11 amino acids" is selected, all divided sequences consist of 11 amino acids). In addition, overlaps between divided amino acid sequences also consist of a fixed number of amino acids. (For example, if an overlap of 3 amino acids is selected, all divided amino acid sequences have an overlap of 3 amino acids at both ends. If no overlap is provided, all divided sequences have no overlap.)

The number of divided amino acid sequences to be connected might depend on the length of the antigen protein. The number of divided amino acid sequences to be connected is not particularly limited. However, connecting as many different divided amino acid sequences as possible is expected to increase the likelihood of including an MHC class I epitope sequence specific for the antigen protein and increase the number of such sequences. The number of divided amino acid sequences to be connected is, for example, 10 or greater, preferably 15 or greater, 20 or greater, 25 or greater, 30 or greater, 35 or greater, 40 or greater, 45 or greater, 50 or greater, or 55 or greater. A connected amino acid sequence thus produced preferably has a high ratio of coverage of the amino acid sequence of the antigen protein as described above, and it is preferred not to place a gap when dividing wherever possible. For example, when the amino acid sequence of an antigen protein is divided into 11-amino acid sequences overlapping with one another by 3 residues, the length of the antigen protein required to ensure 10 divided amino acid sequences is 83 amino acids, and the length of the antigen protein required to ensure 20 divided amino acid sequences is 163 amino acids, Accordingly, it is preferred to select a source antigen protein with a length of, for example, 80 amino acids or longer, preferably 85 amino acids or longer, preferably 100 amino acids or longer, more preferably 150 amino acids or longer, 200 amino acids or longer, 250 amino acids or longer, 300 amino acids or longer, or 350 amino acids or longer, more preferably 400 amino acids or longer. In addition, a connected polypeptide (connected amino acid sequence) has a length of, for example, 100 amino acids or longer, preferably 120 amino acids or longer, preferably 150 amino acids or longer, more preferably 200 amino acids or longer, 250 amino acids or longer, 300 amino acids or longer, 350 amino acids or longer, or 400 amino acids or longer, more preferably 500 amino acids or longer.

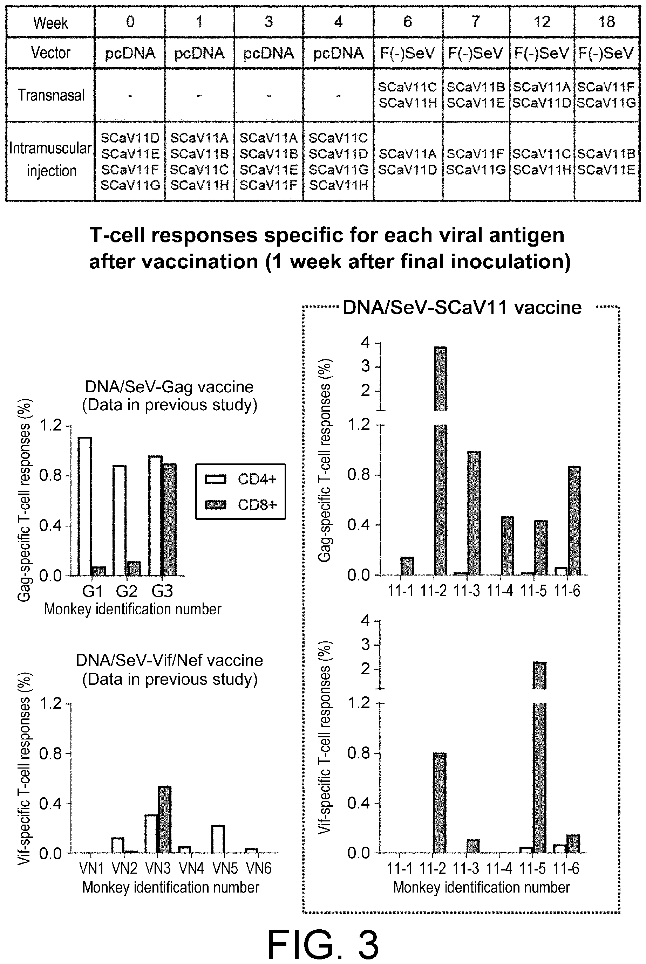

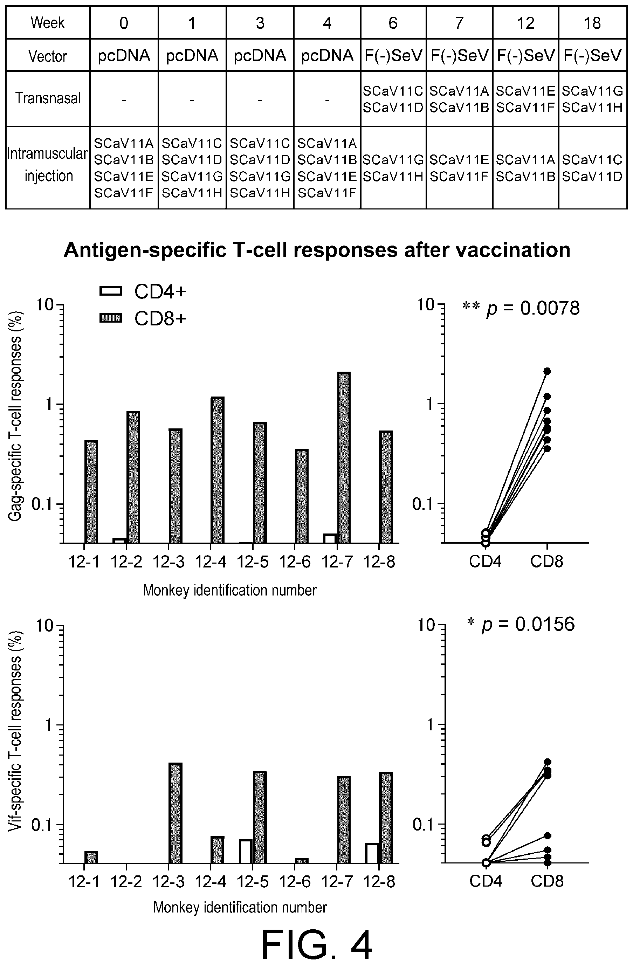

A polypeptide including a connected amino acid sequence is expected to contain a potential MHC class I epitope for a target antigen protein, but not a potential MHC class II epitope for the antigen protein. Inoculation of this polypeptide as an antigen is expected to induce almost no MHC class II-mediated immune responses against the target antigen protein. In fact, as shown in the graphs of Figs. 3 and 4, whereas the frequency of target antigen-specific CD4-positive T cells is significantly increased when the Gag protein or Vif/Nef protein of HIV is simply inoculated as an antigen, it is hardly increased when a polypeptide of the present invention is inoculated as an antigen. Then it has been found that inoculation of the polypeptide of the present invention as an antigen significantly increases the frequency of target antigen-specific CD8-positive T cells. Therefore, the polypeptide of the present invention is useful for selectively inducing MHC class I-mediated immune responses against a target antigen protein.

MHC class I and MHC class II immune responses against a target antigen protein can be measured by known methods. For example, a polypeptide of the present invention or a nucleic acid or vector encoding the polypeptide is inoculated, and peripheral blood mononuclear cells (PBMCs) are collected from the blood. The obtained cells are stimulated with the antigen, and IFN-γ-producing cells are detected to determine the frequency of target antigen-specific T cells.

When a polypeptide of the present invention is used as an antigen, the frequency of target antigen protein-specific CD8-positive T cells is selectively increased. The term "selectively" means that the increase of the frequency of target antigen protein-specific CD8-positive T cells is significantly higher than the increase of the frequency of target antigen protein-specific CD4-positive T cells. The "increase ratio of the frequency of target antigen protein-specific CD8-positive T cells / increase ratio of the frequency of target antigen protein-specific CD4-positive T cells" (CD8 T frequency increase ratio/CD4 T frequency increase ratio) resulting from a polypeptide of the present invention may be, for example, 1.1 or higher, preferably 1.2 or higher, 1.3 or higher, 1.5 or higher, 2 or higher, 3 or higher, 5 or higher, 10 or higher, 15 or higher, 20 or higher, or 30 or higher. Moreover, the value of "CD8 T frequency increase ratio/CD4 T frequency increase ratio" resulting from the polypeptide of the present invention may be, for example, 1.1 or higher, preferably 1.2 or higher, 1.3 or higher, 1.5 or higher, 2 or higher, 3 or higher, 5 or higher, 10 or higher, 15 or higher, 20 or higher, or 30 or higher, as compared to when the original target antigen protein is used as an antigen.

The measurement of cell frequency mentioned above can be performed at an appropriate time on or after 5 days of inoculation, for example, 1 week, 2 weeks, 3 weeks, or 4 weeks after inoculation. Even when inoculation is carried out multiple times, measurement can be performed at an appropriate time. For example, blood can be collected and measured one week after final inoculation.

When a polypeptide of the present invention is inoculated, the value of "frequency of target antigen protein-specific CD8-positive T cells / frequency of target antigen protein-specific CD4-positive T cells" (CD8 T frequency /CD4 T frequency) may be, for example, 1.1 or higher, preferably 1.2 or higher, 1.3 or higher, 1.5 or higher, 2 or higher, 3 or higher, 5 or higher, 10 or higher, 15 or higher, 20 or higher, or 30 or higher, at any time after 5 days of inoculation. Moreover, the value of "CD8 T frequency /CD4 T frequency" resulting from a polypeptide of the present invention may be, for example, 1.1 or higher, preferably 1.2 or higher, 1.3 or higher, 1.5 or higher, 2 or higher, 3 or higher, 5 or higher, 10 or higher, 15 or higher, 20 or higher, or 30 or higher, as compared to when the original target antigen protein is used as an antigen.

A polypeptide including a connected amino acid sequence may include other amino acid sequences as appropriate. For example, a methionine (M) can be added to the beginning of the polypeptide, and a spacer amino acid may be included between the methionine and the connected amino acid sequence. When an alanine (A) is used as a spacer amino acid, the beginning of the polypeptide (N-terminus) may be MA (Met-Ala). To the C-terminus of the polypeptide, a tag, spacer, and such may be added as appropriate. For example, for experimental use, any desired sequence such as H-2K^{d} RT2 epitope (VYYDPSKDLI / SEQ ID NO: 20) can be added to the C-terminus. Such sequences may be added *via* a spacer amino acid, and a further spacer amino acid (*e.g.* Ala) may be added to the C-terminus.

A polypeptide of the present invention can include a connected amino acid sequence prepared from amino acid sequences of more than one antigen protein. For example, connected amino acid sequences prepared separately from two proteins of a certain pathogen can be connected to make one polypeptide. For example, in the Examples, a connected amino acid sequence prepared from the amino acid sequence of the Gag protein of HIV was joined to a connected amino acid sequence prepared from the amino acid sequence of the Vif protein to produce one polypeptide. In such a manner, a polypeptide of the present invention can include a connected amino acid sequence prepared from more than one antigen protein.

When a polypeptide of the present invention is inoculated as an antigen, more than one polypeptide of the present invention can be used in combination. Here, the phrase "used in combination" is not limited to simultaneous use, and may be use of a series of peptides in a serial or sequential manner. As described above, when the amino acid sequence of an antigen protein is divided into, for example, 11-amino acid sequences, there are 11 dividing frames. Therefore, in order to cover all potential MHC class I epitope (CD8-positive T cell epitope) sequences that may exist in the amino acid sequence of the target antigen protein, for example, 11 connected amino acid sequences are required if the divided amino acid sequences have no overlap, or 8 connected amino acid sequences are required if the divided sequences have an overlap of 3 residues. These connected amino acid sequences can be expressed as polypeptides from a single expression vector, or expressed as a single polypeptide in which the connected amino acid sequences are connected together. However, the connected amino acid sequences, which share many common nucleic acid sequences of about several tens of bases, have a risk of homologous recombination. To avoid that, the recombinant expression of the connected amino acid sequences prepared in different frames is preferably performed by expressing them as separate polypeptides from separate vectors. An appropriate combination of polypeptides including these connected amino acid sequences prepared in different frames or nucleic acids or vectors encoding them can cover a wide range of theoretically possible potential MHC class I epitope sequences, and can be inoculated to efficiently induce target-specific CD8-positive T cells.

For example, in "Case 3" above, a combination of 8 polypeptides including connected amino acid sequences prepared in different frames can cover all *(i.e.* 100%) theoretically possible potential MHC class I epitope sequences (a set of 11-amino acid sequences that may be chosen from the amino acid sequence of the antigen protein). In the present invention, this is called a ratio of coverage of the divided sequences of the antigen protein. This coverage ratio corresponds to a ratio of coverage of the potential MHC class I epitopes present in the antigen protein. In "Case 1" above, the ratio of coverage of the divided sequences in one connected amino acid sequence (when divided into 11-residue sequences; this is referred to as a ratio of coverage of the divisional sequences at a window width of 11 amino acids) is 1/11, *i.e.* 9.1%. When *n* connected amino acid sequences in different frames are combined, the coverage ratio is (1/11)^{∗}*n*%. In "Case 3" above, the ratio of coverage of the divided sequences in one connected amino acid sequence (when divided into 11-residue sequences) is 1/8, *i.e.* 12.5%. When *n* connected amino acid sequences in different frames are combined, the coverage ratio is (1/8)^{∗}*n*%. When multiple polypeptides of the present invention are combined, the combination is such that the ratio of coverage of the divided sequences of the antigen protein is, for example, 20% or higher, preferably 25% or higher, more preferably 30% or higher, even more preferably 35% or higher, still more preferably 40% or higher, even more preferably 45% or higher, still more preferably 50% or higher, 60% or higher, 70% or higher, 80% or higher, 90% or higher, 95% or higher, or 100%. This coverage ratio is calculated in accordance with the length of the divided amino acid sequences. The amino acid length *(i.e.* window width) is, for example, 8 amino acids, preferably 9 amino acids, more preferably 10 amino acids, still more preferably 11 amino acids. For example, to achieve 60% or higher coverage of the divided sequences of the antigen protein in "Case 3" above (divided into 11-amino acid sequences with an overlap of 3 residues), 6 connected amino acid sequences in different frames are combined.

For example, polypeptides including connected amino acid sequences of the present invention may be a combination of at least 2, preferably 2, more preferably 4, even more preferably 5, 6, 7, 8, 9, 10, or 11 polypeptides in different dividing frames. These polypeptides may be prepared as separate vaccine compositions or mixed in a single composition.

The present invention also relates to nucleic acids that encode a polypeptide including a connected amino acid sequence of the present invention. Such a nucleic acid is not particularly limited, and may be DNA or RNA. Meanwhile, negative-strand RNA viral vectors, which are described later, are viruses having an antisense single-stranded RNA genome, which encodes proteins in the antisense orientation. Thus, the nucleic acids of the present invention include not only those encoding a polypeptide in the sense strand but also those encoding a polypeptide in the antisense strand. In addition, the nucleic acids may be single-stranded or double-stranded. In designing the nucleotide sequence of a nucleic acid, the codons may be appropriately optimized according to the host for expressing the polypeptide.

A nucleic acid of the present invention may encode other polypeptides as long as it encodes the polypeptide of the present invention. It may also contain other sequences such as a replication origin, promoter, enhancer, terminator, and spacer.

The present invention also provides vectors containing such a nucleic acid. A vector of the present invention is not particularly limited as long as it carries a nucleic acid of the present invention. For example, the vector may be a plasmid vector, phage vector, cosmid, viral vector, artificial chromosome, or such. In particular, the vectors of the present invention include expression vectors. By using an expression vector that can be administered to animals in vivo, a polypeptide of the present invention can be expressed in the animal body to function as a vaccine.

Such vectors include non-viral vectors and viral vectors, including, for example, plasmid vectors, adenoviral vectors, retroviral vectors (including lentiviral vectors), adeno-associated viral vectors, vaccinia virus vectors, cytomegalovirus vectors, and pox virus vectors (Wilson NA. et al., J Virol. 80: 5875-5885, 2006; Hansen SG et al., Nature. 473: 523-527, 2011; Barouch DH. et al., Nature. 482: 89-93, 2012), but are not limited thereto.

In particular, the vectors of the present invention include negative-strand RNA viral vectors. The present inventors' study using a Sendai virus (SeV) vector, which is one of the negative-strand RNA viral vectors, in a simian AIDS model has shown that a SeV vector vaccine expressing a single CD8-positive T cell SIV epitope did not induce SIV-specific CD4-positive T cells but induced effective SIV epitope-specific CD8-positive T cells (Tsukamoto T. et al., J Virol. 83: 9339-9346, 2009; Ishii H. et al., J Virol. 86: 738-745, 2012). Therefore, the use of a negative-strand RNA viral vector to express a polypeptide of the present invention is expected to more highly induce effective antigen-specific CD8-positive T cells selectively while suppressing the induction of antigen-specific CD4-positive T cells as much as possible.

As described above, negative-strand RNA viral vectors are chromosomally non-integrating viral vectors and expressed within the cytosol. Therefore, they have no risk of integrating genes they carry into host chromosomes (nuclear chromosomes). They are therefore highly safe, and also easily removed from infected cells. Negative-strand RNA viral vectors including Sendai virus (SeV) vectors (Matano T. et al., J Exp Med. 199: 1709-1718, 2004; Nyombayire J. et al., J Infect Dis. 215: 95-104, 2017) are useful as vectors capable of inducing effective CD8-positive T cells.

In the present invention, the negative-strand RNA viral vectors include infectious viral particles, and also include viral cores, complexes composed of a viral genome and viral proteins, or complexes composed of a non-infectious viral particle and such, that are capable of expressing a gene they carry when introduced into cells. For example, the ribonucleoprotein (viral core) of a negative-strand RNA virus, which consists of a viral genome and negative-strand RNA virus proteins binding to it (*e.g.* NP, P, and L proteins), can express a transgene intracellularly when introduced into cells (WO00/70055). The introduction into cells may be performed using a transfection agent and such, as appropriate. Such ribonucleoproteins (RNPs) are also included in the negative-strand RNA viral vectors in the present invention. Preferably, a negative-strand RNA viral vector in the present invention is a particle in which the aforementioned RNP is enclosed by a biological membrane derived from the cell membrane.

Negative-strand RNA viral vectors used in the present invention particularly include paramyxovirus vectors. The paramyxovirus refers to a virus belonging to the family *Paramyxoviridae* or a derivative thereof. *Paramyxoviridae* includes the subfamilies *Paramyxovirinae* (including the genera *Respirovirus* (also called *Paramyxovirus*), *Rubulavirus,* and *Morbillivirus*) and *Pneumovirinae* (including the genera *Pneumovirus* and *Metapneumovirus*)*.* The viruses belonging to the family *Paramyxoviridae* specifically include Sendai virus, Newcastle disease virus, mumps virus, measles virus, RS virus (respiratory syncytial virus), (rinderpest virus), distemper virus, simian parainfluenza virus (SV5), human parainfluenza virus types 1, 2, and 3. More specifically, those viruses include, for example, Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MeV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV). Rhabdovirus includes vesicular stomatitis virus and rabies virus, which belong to the family *Rhabdoviridae.*

As described above, the genomic RNA of negative-strand RNA viruses is a negative strand. Their protein amino acid sequences are encoded in an antigenome having a sequence complementary to the genomic RNA. In the present invention, both genome and antigenome may be referred to as genome for the sake of convenience.

In the present invention, a viral vector is preferably a virus belonging to the subfamily *Paramyxovirinae* (including the genera *Respirovirus, Rubulavirus,* and *Morbillivirus*) or a derivative thereof, more preferably a virus belonging to the genus *Respirovirus* (also called *Paramyxovirus*) or a derivative thereof. The derivatives include a virus whose viral genes have been altered, and a virus which have been chemically modified, without impairing the gene transfer ability of the virus. Viruses of the genus *Respirovirus* to which the present invention can be applied include, for example, human parainfluenza virus type 1 (HPIV-1), human parainfluenza virus type 3 (HPIV-3), bovine parainfluenza virus type 3 (BPIV-3), Sendai virus (also called murine parainfluenza virus type 1), measles virus, simian parainfluenza virus (SV5), and simian parainfluenza virus type 10 (SPIV-10). In the present invention, the most preferred paramyxovirus is Sendai virus.

A paramyxovirus in general contains within its envelope a complex consisting of RNA and proteins (ribonucleoprotein; RNP). The RNA contained in the RNP is (-)strand (negative-strand), single-stranded RNA, which is a genome of negative-strand RNA virus. This single-stranded RNA binds NP protein, P protein, and L protein to form the RNP The RNA contained in this RNP serves as a template for transcription and replication of the viral genome (Lamb, R.A., and D. Kolakofsky, 1996, Paramyxoviridae: The viruses and their replication. pp. 1177-1204. In Fields Virology, 3rd edn. Fields, B. N., D. M. Knipe, and P. M. Howley et al., (ed.), Raven Press, New York, N. Y).

A viral vector may be derived from a virus of a natural strain, wild-type strain, mutant strain, laboratory-passaged strain, and artificially-established strain, and the like. For Sendai virus, examples include Z strain but are not limited thereto (Medical Journal of Osaka University Vol.6, No.1, March 1955 p1-15). For example, a wild-type virus with a mutation or deficiency in any of its genes may be used. For example, a virus that is deficient in at least one of the genes encoding its envelope protein or coat protein or contains a mutation suppressing the expression thereof such as a stop codon mutation can suitably be used. Such viruses that do not express the envelope protein are, for example, capable of replicating the genome but not capable of forming infectious viral particles in cells they have infected. Such propagation-deficient viruses are particularly suitable as highly safe vectors. For example, a virus that does not encode in its genome one or both of the envelope protein (spike protein) genes F and HN can be used (WO00/70055 and WO00/70070; Li, H.-O. et al., J. Virol. 74(14) 6564-6569 (2000)). A virus can replicate its genome in cells it has infected as long as the genomic RNA encodes at least proteins necessary for genome replication (*e.g.* N, P, and L proteins). To produce envelope protein-deficient, infectious viral particles, for example, the deficient gene product or a protein that can complement it is exogenously supplied in virus-producing cells (WO00/70055 and WO00/70070; Li, H.-O. et al., J. Virol. 74(14) 6564-6569 (2000)). On the other hand, non-infectious viral particles can be recovered by not complementing the deficient viral protein at all (WO00/70070).

In producing a virus of the present invention, it is also preferred to use a virus carrying a mutant viral protein gene. For example, there are a large number of known mutations including attenuating mutations and temperature-sensitive mutations for viral structural proteins (NP, M) and RNA synthase (P, L). Paramyxovirus vectors and such containing these mutant protein genes can suitably be used according to the purpose in the present invention.

Viral vectors containing a nucleic acid encoding a polypeptide of the present invention can be constructed using known methods (WO97/16539; WO97/16538; WO00/70070; WO01/18223; WO2005/071092; Hasan, MK et al., J Gen Virol 78:2813-2820, 1997; Kato A et al., EMBO J 16: 578-587, 1997; Yu D et al., Genes Cells 2: 457-466, 1997; Kato A et al, Genes Cells 1; 569-579, 1996; Tokusumi T et al., Virus Res 86: 33-38, 2002; Li HO et al., J Virol 74: 6564-6569, 2000).

The present invention also provides a composition comprising a polypeptide of the present invention or a nucleic acid or a vector encoding the polypeptide. The composition may contain a desired carrier and/or vehicle. The carriers and vehicles include desired pharmaceutically acceptable carriers and vehicles including, for example, sterile water, physiological saline, phosphate buffered saline (PBS), buffers, and culture fluids. In addition, glycols, glycerol, oils such as olive oil, and organic esters may also be added. Additives such as suspending liquids, emulsifiers, diluents, and excipients may be mixed as appropriate for formulation. Methods of formulation and additives that can be used are well-known in the field of pharmaceutical formulation. The forms of formulation are not particularly limited, and include, for example, injections, inhalants, and capsules. Furthermore, the present invention also relates to vaccine formulations comprising a polypeptide of the present invention or a nucleic acid or a vector encoding the polypeptide. The compositions or vaccine formulations of the present invention are useful for selectively inducing CD8-positive T cells specific for a target antigen protein while suppressing the induction of CD4-positive T cells specific for the antigen protein. The compositions or vaccine formulations of the present invention can be prepared, for example, as a composition containing a polypeptide of the present invention or a nucleic acid or vector encoding the polypeptide, and a desired carrier. The compositions or vaccine formulations of the present invention can be prepared as liposomes such as HVJ liposomes. In addition, the compositions or vaccine formulations of the present invention may further contain a desired adjuvant. Adjuvants include, for example, oil adjuvants and aluminum adjuvants, and more specifically include alum (aluminum salt), MF59 (oil emulsion), and Montanides (such as Montanide ISA 51VG; oil emulsion).

A composition or vaccine formulation of the present invention can contain one or more polypeptides of the present invention. As described above, a combination of polypeptides of the present invention prepared from the amino acid sequence of one antigen protein in different dividing frames can effectively induce target antigen-specific CD8-positive T cells. For example, a composition or vaccine formulation of the present invention may be for combined use of 2 or more, 3 or more, 4 or more, 5 or more, 6 or more, 7 or more, 8 or more, 9 or more, 10 or more, or 11 or more polypeptides of the present invention that target one antigen protein but are prepared in different dividing frames, or alternatively the composition or vaccine formulation may contain those polypeptides. When multiple polypeptides of the present invention prepared in different dividing frames are combined, the combination is such that the ratio of coverage of the divided sequences of the antigen protein is, for example, 20% or higher, preferably 25% or higher, more preferably 30% or higher, even more preferably 35% or higher, still more preferably 40% or higher, even more preferably 45% or higher, still more preferably 50% or higher, 60% or higher, 70% or higher, 80% or higher, 90% or higher, 95% or higher, or 100%. Furthermore, a composition or vaccine formulation of the present invention may be for use in combination with polypeptides of the present invention targeting a different antigen protein, or alternatively the composition or vaccine formulation may further contain those polypeptide.

When a vaccine formulation of the present invention is used, the mode of inoculation thereof is not particularly limited. For example, the vaccine formulation can be used in single or multiple inoculations. In multiple inoculations, the vaccine of the present invention may be inoculated multiple times, or alternatively may be used in combination with other types of vaccine. For example, in performing multiple injections, it may be beneficial to change the polypeptide or combination of peptides to inoculate, rather than repeating the inoculation of the same polypeptide or combination. It may also be beneficial to change the administration route or use more than one administration route for inoculation. Specifically, in the case where all theoretically possible divided amino acid sequences (*i.e*. potential MHC class I epitope sequences) can be covered by 8 polypeptides, for example, 4 polypeptides can be inoculated at a time, and the combination of 4 polypeptides can be changed in each inoculation (see Example 4 and Fig. 3). It is also possible to perform primary inoculation or the first few inoculations using a non-viral vector (*e.g.* polypeptide or DNA vector) and subsequent inoculations using a viral vector encoding the polypeptide of the present invention. The viral vector to be used is not particularly limited. For example, paramyxovirus vectors such as Sendai virus vectors may be suitably used.

A polypeptide, nucleic acid, and vector of the present invention can be used in combination with another antigen or a nucleic acid or vector encoding that antigen. For example, primary inoculation or the first few inoculations can be performed using a target antigen that has not been divided like the polypeptide of the present invention, and then the polypeptide, nucleic acid or vector of the present invention can be inoculated in booster inoculations (see Examples 4 and 5). Primary inoculation can be performed, for example, using a DNA vector encoding a target antigen that has not been divided like the polypeptide of the present invention, but is not limited thereto.

When a composition or vaccine formulation of the present invention is inoculated into an animal, its dose can be appropriately determined according to the disease, patient's weight, age, sex, and symptoms, purpose of administration, form of administered composition, administration method, and the like. The route of administration can be appropriately selected, and includes, for example, transnasal administration, intraperitoneal administration, intramuscular administration, and local administration to lesions of infection, tumor, and such, but is not limited thereto. The dose may be appropriately adjusted according to the subject animal, site of administration, and number of administrations. For example, the dose may be from 1 ng/kg to 1000 mg/kg, from 5 ng/kg to 800 mg/kg, from 10 ng/kg to 500 mg/kg, from 0.1 mg/kg to 400 mg/kg, from 0.2 mg/kg to 300 mg/kg, from 0.5 mg/kg to 200 mg/kg, or from 1 mg/kg to 100 mg/kg, but is not limited thereto. In the case of an viral vector, for example, the dose may be from 1 x 10⁴ to 1 x 10¹⁵ CIU/kg, from 1 x 10⁵ to 1 x 10¹⁴ CIU/kg, from 1 x 10⁶ to 1 x 10¹³ CIU/kg, from 1 x 10⁷ to 1 x 10¹² CIU/kg, from 1 x 10⁸ to 5 x 10¹¹ CIU/kg, from 1 x 10⁹ to 5 x 10¹¹ CIU/kg, or from 1 x 10¹⁰ to 1 x 10¹¹ CIU/kg; or may be from 1 x 10⁶ to 1 x 10¹⁷ particles/kg, from 1 x 10⁷ to 1 x 10¹⁶ particles/kg, from 1 x 10⁸ to 1 x 10¹⁵ particles/kg, from 1 x 10⁹ to 1 x 10¹⁴ particles/kg, from 1 x 10¹⁰ to 1 x 10¹³ particles/kg, from 1 x 10¹¹ to 5 x 10¹² particles/kg, or from 5 x 10¹¹ to 5 x 10¹² particles/kg, but is not limited thereto.

Subjects to which a composition or vaccine formulation of the present invention is administered are not particularly limited, but preferably are mammals (including human and non-human mammals). Specifically, the subjects include human, non-human primates such as monkeys, rodents such as mice and rats, rabbits, goats, sheep, pigs, cows, dogs, cats, and all other mammals.

A composition or vaccine formulation of the present invention can be used in combination with other pharmaceuticals. For example, when a polypeptide of the present invention designed against a tumor antigen is used, the composition or vaccine formulation may be used in combination with other anticancer agents. When a polypeptide of the present invention designed against an infectious disease is used, the composition or vaccine formulation may be used in combination with other drugs for that infectious disease.

### [Examples]

Herein below, the present invention will be specifically described with reference to Examples, but it is not to be construed as being limited thereto. All cited documents and other references herein are incorporated as part of this specification.

### [Example 1] Construction of plasmid carrying SCaV11 antigen gene

The Gag CA and Vif proteins of SIVmac239 (GenBank Accession No. M33262) were used as target antigens to design a TCT11 antigen (referred to as SCaV11) for evaluation in the SIVmac239-infected monkey AIDS model. The amino acid sequences of the Gag CA protein (amino acid sequence Accession : AAA47632.1 (SEQ ID NO: 21)) and Vif protein (amino acid sequence Accession : AAA47634.1 (SEQ ID NO: 22)) of SIVmac239 were fragmented into 11-mer peptides with an overlap of 3 amino acids with one another. These peptides were rearranged in a different order and connected in tandem using alanine as a spacer (SCaV11)(Fig. 1). The 3-amino acid overlap was for preventing homologous recombination. In a similar manner, a total of 8 tandemly-connected antigens (SCaV11A to pSCaV11H) were designed, for each of which the starting amino acid position of the peptides in the target antigen region was shifted by one amino acid (SEQ ID NOs: 23 to 30, in order). Next, the nucleotide sequences for these antigens were codon-optimized for human, and mutations for preventing homologous recombination were introduced into the sequences. The entire genes were then chemically synthesized (Eurofins Genomics), and inserted into plasmids. These plasmids were named pSCaV111A to pSCaV11H (SEQ ID NOs: 31-38, in order)

### [Example 2] Construction of Sendai virus (SeV) vector carrying SCaV11 antigen gene (1) Construction of plasmids for producing F-deficient Sendai viruses carrying SCaV11 antigen genes

PCR was performed on the plasmid carrying the SCaV11A antigen gene as a template, using primers Not1_SCaV11A_N
(5'-ATATgcggccgcgacgccaccATGGCCTACCCTGTGCAGCAG-3' (SEQ ID NO: 39)) and SCaV11A_EIS_Not1_C
(5'-ATATGCGGCCGCgatgaactttcaccctaagtttttcttactacggTCAGGCTTTGCCTCCCCTCTGC-3'
(SEQ ID NO: 40)), and KOD-Plus-Ver.2 kit, under the following conditions: 94°C for 2 min; 30 cycles of 98°C for 10 sec, 55°C for 30 sec, and 68°C for 2.5 min; react at 68°C for 7 min; and keep at 4°C. The amplified SCaV11A fragment was separated by agarose gel electrophoresis, and then purified using NucleoSpin Gel and PCR Clean-up kit (Takara Bio). In the above primer sequences, the upper-case letters represent a sequence of the SCaV11 antigen gene, and the lower-case letters represent a sequence of the SeV vector (the same applies hereinafter).

Next, the above SCaV11A fragment treated with *Not*I (having a *Not*I site on both ends) was ligated into the *Not*I cleavage site of plasmid pSeV18+/ΔF (WO00/070070), which encodes an F gene-deficient Sendai virus vector. The plasmid was used for transformation of E. coli followed by cloning. Sequencing was performed to select a clone with the correct nucleotide sequence, and thereby plasmid pSeV18+SCaV11A/ΔF was obtained.

Similarly, PCR was performed on the plasmid carrying the SCaV11B antigen as a template using primers Not1_SCaV11B_N
(5'-ATATgcggccgcgacgccaccATGGCCCCTGTGCAGCAGATCG-3' (SEQ ID NO: 41)) and SCaV11B_EIS_Not1_C
(5'-ATATGCGGCCGCgatgaactttcaccctaagtttttcttactacggTCAGGCGGGCTTCCCTCCCCTC-3' (SEQ ID NO: 42)), and the amplified fragment was inserted into the *Not*I site of plasmid pSeV18+/ΔF to obtain plasmid pSeV18+SCaV11B/ΔF.

Similarly, PCR was performed on the plasmid carrying the SCaV11C antigen as a template using primers Not1_SCaV11C_N
(5'-ATATgcggccgcgacgccaccATGGCCGTGCAGCAGATCGGAG-3' (SEQ ID NO: 43)) and SCaV11C_EIS_Not1_C
(5'-ATATGCGGCCGCgatgaactttcaccctaagtttttcttactacggTCAAGCAGGAGGTTTCCCTCCCC-3 ' (SEQ ID NO: 44)), and the amplified fragment was inserted into the *Not*I site of plasmid pSeV18+/ΔF to obtain plasmid pSeV18+SCaV11C/ΔF.

Similarly, PCR was performed on the plasmid carrying the SCaV11D antigen as a template using primers Not1_SCaV11D_N
(5'-ATATgcggccgcgacgccaccATGGCCCAGCAGATCGGAGGC-3' (SEQ ID NO: 45)) and SCaV11D_EIS_Not1_C
(5'-ATATGCGGCCGCgatgaactttcaccctaagtttttcttactacggTCAGGCTGTTGGGGG TTTCCCTC-3' (SEQ ID NO: 46)), and the amplified fragment was inserted into the *Not*I site of plasmid pSeV18+/ΔF to obtain plasmid pSeV18+SCaV11D/ΔF.

Similarly, PCR was performed on the plasmid carrying the SCaV11E antigen as a template using primers Not1_SCaV11E_N
(5'-ATATgcggccgcgacgccaccATGGCCCAGATCGGAGGCAATTATG-3' (SEQ ID NO: 47)) and SCaV11E_EIS_Not1_C
(5'-ATATGCGGCCGCgatgaactttcaccctaagtttttcttactacggTCAGGCCTTGGTAGGGGGTTTCC-3' (SEQ ID NO: 48)), and the amplified fragment was inserted into the *Not*I site of plasmid pSeV18+/ΔF to obtain plasmid pSeV18+SCaV11E/ΔF.

Similarly, PCR was performed on the plasmid carrying the SCaV11F antigen as a template using primers Not1_SCaV11F_N
(5'-ATATgcggccgcgacgccaccATGGCCATCGGAGGCAATTATG-3' (SEQ ID NO: 49)) and SCaV11F_EIS_*Not*1_C
(5'-ATATGCGGCCGCgatgaactttcaccctaagtttttcttactacggTCAGGCGCCTTTTGTAGGGGG-3' (SEQ ID NO: 50), and the amplified fragment was inserted into the *Not*I site of plasmid pSeV18+/ΔF to obtain plasmid pSeV18+SCaV11F/ΔF.

Similarly, PCR was performed on the plasmid carrying the SCaV11G antigen as a template using primers Not1_SCaV11G_N
(5'-ATATgcggccgcgacgccaccATGGCCGGAGGCAATTATGTG-3' (SEQ ID NO: 51)) and SCaV11G_EIS_Not1_C
(5'-ATATGCGGCCGCgatgaactttcaccctaagtttttcttactacggTCAGGCGGCGCCCTTTGTAGGGG-3 ' (SEQ ID NO: 52)), and the amplified fragment was inserted into the *Not*I site of plasmid pSeV18+/ΔF to obtain plasmid pSeV18+SCaV11G/ΔF.

Similarly, PCR was performed on the plasmid carrying the SCaV11H antigen as a template using primers Not1_SCaV11H_N
(5'-ATATgcggccgcgacgccaccATGGCCGGAGGCAATTATGTG-3' (SEQ ID NO: 53)) and SCaV11H_EIS_Not1_C
(5'-ATATGCGGCCGCgatgaactttcaccctaagtttttcttactacggTCAGGCGGCGCCCTTTGTAGGGG-3 ' (SEQ ID NO: 54)), and the amplified fragment was inserted into the *Not*I site of plasmid pSeV18+/ΔF to obtain plasmid pSeV18+SCaV11H/ΔF.

### (2) Production (reconstitution) and amplification of F-deficient Sendai virus vectors carrying SCAV11 antigen genes

From the plasmids produced as described above for producing SCaV11 antigen gene-carrying F-deficient Sendai viruses, namely, pSeV18+SCaVIIA/ΔF to pSeV18+SCaV11H/ΔF, the SCaV11 antigen gene-carrying F-deficient Sendai viruses were produced (reconstituted) and amplified by a known method (for example, WO2005/071092). The resulting viruses were named SeV18+SCaV11A/ΔF, SeV18+SCaV11B/ΔF, SeV18+SCaV11C/ΔF, SeV18+SCaV11D/ΔF, SeV18+SCaV11E/ΔF, SeV18+SCaV11F/ΔF, SeV18+SCaV11G/ΔF, and SeV18+SCaV11H/ΔF, respectively.

### [Example 3] Inoculation test of SIV CA-Vif TCT11 antigen-expressing vaccines into SIV controllers (SIV replication-controlled monkeys)

Rhesus monkeys that had controlled SIV replication (SIV controllers) after inoculation of a single epitope (Gag CA)-expressing vaccine followed by transvenous inoculation of SIVmac239 were inoculated with the instant SCaV11-expressing Sendai virus (SeV) vectors during their chronic phase, and examined for induced T-cell responses specific for SIV Gag and Vif antigens.

The F-deficient Sendai virus vectors expressing SCaV11A, SCaV11B, SCaV11F, and SCaV11H(SeV18+SCaV11A/ΔF, SeV18+SCaV11B/ΔF, SeV18+SCaV11F/ΔF, and SeV18+SCaV11H/ΔF; 6 x 10⁹ CIU each) were inoculated transnasally and intramuscularly. Peripheral blood mononuclear cells (PBMCs) were isolated from the blood before vaccination and after one week of vaccination, and analyzed for T-cell responses specific for SIV Gag and Vif antigens. Specifically, the cells were challenged with a pool of overlapping peptides spanning the Gag and Vif regions of SIVmac239, and the frequency of SIV Gag/Vif antigen-specific T cells was determined by detection of IFN-γ-producing cells by intracellular cytokine staining using a flow cytometer. As a result, the frequency of Gag/Vif antigen-specific CD8-positive T cells after vaccination was increased 10-fold or more as compared to that before vaccination; however, the frequency of Gag/Vif antigen-specific CD4-positive T cells was not changed by vaccination (Fig. 2). This result demonstrated that the SCaV11 antigen-expressing SeV vector vaccines induced SIV Gag/Vif antigen-specific CD8-positive T-cell responses in a selective manner.

### [Example 4] Inoculation test of SCaV11 vaccines in uninfected monkeys

Six uninfected rhesus monkeys were inoculated with the SCaV11 antigen-expressing vaccines and examined for induced SIV antigen-specific T-cell responses.

The 6 monkeys were intramuscularly injected with the plasmid DNA vaccines expressing antigens SCaV11A to SCaV11H (8 antigens) (pcDNA-SCaV11A to pcDNA-SCaV11H, respectively, 5 mg each) twice for each vaccine. The monkeys were then inoculated with the F-deficient SeV vector vaccines expressing antigens SCaV11A to SCaV11H (8 antigens) (SeV18+SCaV11A/ΔF to SeV18+SCaV11H/ΔF, 6 x 10⁹ CIU each) transnasally and intramuscularly once for each vaccine (Fig. 3). PBMCs were isolated from the blood after one week of the final vaccination, and analyzed for SIV Gag/Vif antigen-specific T-cell responses by the same method as in Example 3.

It had been previously reported that vaccination with DNA/SeV vectors expressing SIV Gag antigen or Vif/Nef antigen effectively induced not only Gag/Vif antigen-specific CD8-positive T-cell responses but also Gag/Vif-specific CD4-positive T-cell responses (Iwamoto N. et al., J Virol. 88:425-433, 2014). On the other hand, the vaccination with the instant SCaV11 antigen-expressing DNA/SeV vectors, while inducing very efficient Gag/Vif antigen-specific CD8-positive T-cell responses, resulted in undetectable or very low levels of Gag/Vif-specific CD4-positive T-cell responses (Fig. 3). These results demonstrated that the DNA prime/SeV vector vaccines expressing SCaV11 antigens induced almost no SIV Gag/Vif antigen-specific CD4-positive T-cell responses, and selectively induced Gag/Vif antigen-specific CD8-positive T cells efficiently.

### [Example 5] Inoculation test 2 of SCaV11 vaccines in uninfected monkeys

Eight uninfected rhesus monkeys were inoculated with SCaV11 antigen-expressing vaccines and examined for induced SIV antigen-specific T-cell responses.

The 8 monkeys were intramuscularly injected with the plasmid DNA vaccines expressing antigens SCaV11A to SCaV11H (8 antigens) (pcDNA-SCaV11A to pcDNA-SCaV11H, respectively, 5 mg each) twice for each vaccine. The monkeys were then inoculated with the F-deficient SeV vector vaccines expressing antigens SCaV11A to SCaV11H (8 antigens) (SeV18+SCaV11A/ΔF to SeV18+SCaV11H/ΔF, 1 x 10⁹ CIU each) transnasally and intramuscularly once for each vaccine (Fig. 4). PBMCs were isolated from the blood after one week of the final vaccination, and analyzed for SIV Gag/Vif antigen-specific T-cell responses by the same method as in Example 3. In addition, in order to assess the immune induction ability in the lymph node, lymph node biopsy was performed 2 weeks after the third SeV vector vaccination, and SIV Gag/Vif antigen-specific T-cell responses were analyzed in the same manner.

The SCaV11 antigen-expressing DNA/SeV vector vaccination induced very efficient Gag/Vif antigen-specific CD8-positive T-cell responses, but resulted in undetectable or very low levels of Gag/Vif-specific CD4-positive T-cell responses (Fig. 4). Comparison between antigen-specific CD4-positive T-cell responses and CD8-positive T-cell responses also showed that the frequency of antigen-specific CD8-positive T cells was significantly higher than the frequency of antigen-specific CD4-positive T cells for both Gag- and Vif-specific T cells (Gag; p = 0.0078, Vif; p = 0.0156 by Wilcoxon matched-pairs signed rank test). These results confirmed the reproducibility of the selective induction of Gag/Vif-specific CD8-positive T-cell responses by the SCaV11 antigen-expressing vaccines, and verified and confirmed the ability of the target 11-mer connected antigen TCT11 vaccine to induce antigen-specific CD8-positive T-cell responses selectively.

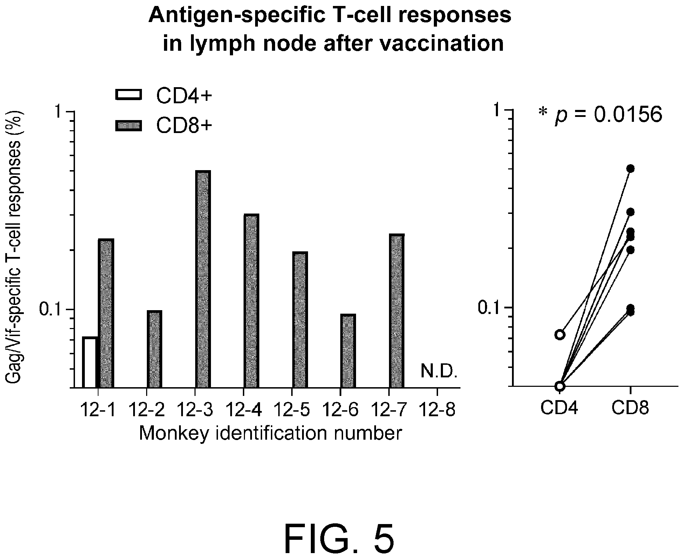

The result of analyzing antigen-specific T-cell responses in the post-vaccination lymph node showed that Gag/Vif-specific CD8-positive T-cell responses were also selectively induced in the lymph node, and Gag/Vif-specific CD4-positive T-cell responses were below the detection limit except for one animal (Fig. 5). As the lymph node is one of the major tissues in which HIV and SIV proliferate, the selective antigen-specific CD8-positive T-cell response in the lymph node may contribute to the replication control of HIV and SIV.

### [Industrial Applicability]

In antigen optimization studies aiming to induce effective CD8-positive T cells, the analyses of HIV-infected people and simian AIDS models have shown that CD8-positive T cell responses targeting Gag and Vif antigens are able to suppress virus replication potently. Meanwhile, based on the idea that antigen regions relatively conserved among various HIV strains may be CD8-positive T cell targets in which the selection of escape mutations is unlikely to occur, an antigen consisting of these conserved regions connected together has been designed (Letourneau S. et al., PLoS One. 2:e984, 2007). Moreover, an antigen in which regions including CD8-positive T cell targets associated with low viral loads (highly capable of suppressing HIV replication) are connected has also been designed (Mothe B. et al., J Transl Med. 9:208, 2011). These antigens all have Gag CA and Vif regions as the main regions. However, there has so far been no antigen designed from the viewpoint of inducing effective HIV antigen-specific CD8-positive T cells selectively while suppressing the induction of HIV antigen-specific CD4-positive T cells as much as possible. Therefore, the novelty, originality, and superiority of the present invention is extremely high. Moreover, the present antigen design theory is also applicable to the design of the above-mentioned antigens consisting of conserved regions connected together or potent HIV replication-suppressing CD8-positive T cell targets connected together. The present invention is expected to pave the way for a more effective vaccine therapy against AIDS.

## Claims

1. A polypeptide comprising multiple peptides connected together, wherein each of the multiple peptides has an amino acid sequence of eight to twelve residues included in the amino acid sequence of an antigen protein.

2. The polypeptide of claim 1, wherein the eight- to twelve-residue peptides are connected in an order different from that in the antigen protein.

3. The polypeptide of claim 1 or 2, which does not substantially comprise a partial amino acid sequence of 13 or more consecutive residues in the antigen protein.

4. The polypeptide of any one of claims 1 to 3, wherein the amino acid sequences of the multiple peptides optionally comprise an overlap.

5. The polypeptide of claim 4, wherein the overlap consists of one to four residues.

6. The polypeptide of any one of claims 1 to 5, wherein each of the connection sites optionally comprises a spacer.

7. The polypeptide of claim 6, wherein the spacer consists of one to four amino acid residues.

8. The polypeptide of any one of claims 1 to 7, wherein at least 20 eight- to twelve-residue peptides are connected together.

9. A nucleic acid encoding the polypeptide of any one of claims 1 to 8.

10. A vector comprising the nucleic acid of claim 9.

11. The vector of claim 10, which is a Sendai virus vector.

12. A vaccine comprising the polypeptide of any one of claims 1 to 8, a nucleic acid encoding the polypeptide, or a vector comprising the nucleic acid.

13. The vaccine of claim 12, wherein the antigen protein is derived from an antigen protein of a human immunodeficiency virus.

14. A method for selectively inducing CD8-positive T cells specific for a target antigen, which comprises inoculating the vaccine of claim 12 or 13.

15. A method for producing the polypeptide of claim 1 or a nucleic acid encoding the polypeptide, which comprises:
(i) dividing an amino acid sequence encoding an antigen protein into amino acid sequences of eight to twelve residues, wherein the divided amino acid sequences may or may not overlap with one another;
(ii) connecting the divided amino acid sequences in such a way as not to become the same as the amino acid sequence of the antigen protein, wherein a spacer may or may not be inserted in each of the connection sites of the divided amino acid sequences; and
(iii) obtaining a polypeptide comprising an amino acid sequence resulting from step (ii) or a nucleic acid encoding the polypeptide.
